# DEMANDE DE BREVET EUROPEEN

(11) **EP 1 514 532 A1**
(43) Date de publication de la demande: **16.03.2005**
(21) Numéro de dépôt: 04104402.5
(22) Date de dépôt: 13.09.2004
(51) Int. Cl.: A61K 7/00, C08F 293/00

(54) **Copolymères éthyléniques séquencés comprenant une séquence vinyllactame, compositions cosmétiques ou pharmaceutiques les contenant, et utilisation de ces copolymères en cosmétique**

(30) Priorité: 15.09.2003 FR 0350538
(71) Demandeur: L'OREAL, 75008 Paris (FR)
(72) Inventeur: Mougin, Nathalie, 75011, PARIS (FR)
(74) Mandataire: Poulin, Gérard

(57) **Abrégé**

Copolymère éthylénique, séquencé, linéaire comprenant :
- au moins une séquence A susceptible d'être préparée à partir de monomères, constitués de 52 à 99% en poids d'un monomère éthylénique à cycle lactame ;
- et de 1 à 48% en poids d'au moins un monomère hydrophile ionique ;
- et au moins une séquence B susceptible d'être préparée à partir de monomères ne comportant pas de monomère éthylénique à cycle lactame ou comportant une proportion minoritaire de celui-ci.

Composition cosmétique ou pharmaceutique en particulier composition capillaire comprenant ledit copolymère.

Procédé cosmétique de maquillage ou de soin des matières kératiniques comprenant l'application sur les matières kératiniques d'une telle composition.

Utilisation des copolymères pour améliorer la tenue de la coiffure, sans collant, notamment au toucher, d'une laque pour cheveux ; utilisation des copolymères pour améliorer l'élimination par un shampooing d'une composition capillaire telle qu'une laque ; utilisation des copolymères pour améliorer l'adhérence et la résistance à l'usure, sans collant, notamment au toucher d'un vernis à ongles ; utilisation des copolymères pour optimiser l'adhérence sur la peau et le confort d'une composition de maquillage ; utilisation des copolymères pour diminuer le collant, notamment au toucher, d'une composition cosmétique, en particulier dans des conditions d'humidité élevée par exemple pour des HR de 50% à 100% ; utilisation des copolymères dans une composition cosmétique telle qu'une composition de soin ou de maquillage pour masquer les rides, qui donne à la peau un aspect lissé, sans tiraillement.

## Description

### DOMAINE TECHNIQUE

La présente invention a trait à de nouveaux polymères de structure spécifique de type copolymère éthylénique séquencé comprenant une séquence vinyllactame.

La présente invention concerne, en outre, une composition, notamment cosmétique ou pharmaceutique en particulier une composition capillaire, comprenant ledit polymère de structure spécifique.

L'invention a également trait à l'utilisation de ces polymères en cosmétique pour le traitement des matières kératiniques, notamment de la peau, des ongles ou des cheveux.

Précisons que par « séquence vinyllactame », on entend une séquence comprenant un motif lactame, par exemple des dérivés de lactames, en d'autres termes, de manière générale, une séquence susceptible d'être préparée par polymérisation d'un monomère à cycle lactame.

De nombreuses compositions cosmétiques, et en particulier les compositions capillaires, dites compositions de "hair styling", qui se présentent sous la forme d'aérosols ("sprays") de gels, de mousses ou de shampooings contiennent des résines ou polymères.

Il s'agit, en particulier, de polymères acryliques ayant des températures de transition vitreuse (Tg) élevées, tels que ceux décrits dans le document FR-A-2 439 798.

De tels polymères, apportent, notamment en coiffage, un maintien de la chevelure, mais ils présentent l'inconvénient d'une trop grande friabilité, ce qui ne permet pas une bonne tenue dans le temps de la chevelure.

Dans le cas des vernis, les polymères existants ne résistent pas aux chocs.

Pour résoudre les problèmes posés par ces polymères, on utilise, en outre, dans les compositions cosmétiques, des plastifiants, afin d'abaisser la température de transition vitreuse. Mais, alors, les polymères tendent à présenter des effets de « collants » ou, dans le cas du coiffage ne permettent pas de fixation « ultra-forte ».

En outre, lorsque les cheveux sont coiffés, nombreux sont les polymères de coiffage ("hair styling") existant qui forment des particules blanches, ce qui n'est pas acceptable, en particulier lorsque les cheveux sont bruns et/ou épais. D'autres inconvénients présentés par les polymères utilisés à l'heure actuelle sont leur incompatibilité avec les propulseurs pour aérosols existant.

Une élimination au shampooing de plus en plus facile, et avec tous types de shampooings est également recherchée.

On connaît aussi, par ailleurs, dans le domaine de la cosmétique des polymères ou copolymères non ioniques à base de vinyllactames par exemple de vinylpyrrolidone, et en particulier les copolymères vinylpyrrolidone / acétate de vinyle.

Le principal défaut de ces copolymères est, de nouveau, leur difficulté à s'éliminer sous l'action des shampooings

Un autre défaut de ces polymères, à base de vinyllactames, est leur forte hygroscopie, qui conduit à leur donner en présence de l'humidité ambiante, un fort caractère collant.

Ainsi, le document EP-A-1 002 811 de BASF décrit des polymères greffés solubles dans l'eau ou dispersibles dans l'eau obtenus par polymérisation radicalaire de monomères essentiellement acryliques, et d'un prépolymère polymérisable à base de vinyllactame par exemple de vinylpyrrolidone ou de vinylcaprolactame.

Ces polymères sont utilisés notamment dans des compositions capillaires.

Le document US-A-6 193 961 de ISP décrit un terpolymère homogène de N-vinyllactame de préférence de N-vinylpyrrolidone ou de N-vinyl-caprolactame, d'acrylate de diméthylaminoalkyle ou de diméthylaminoakylacrylamide et d'un monomère polysiloxane.

Ces terpolymères sont utilisés dans des compositions cosmétiques et de soins par exemple des compositions cosmétiques telles que des gels coiffants ou des mousses.

Dans ce document sont cités de nombreux autres brevets mentionnant l'utilisation de polymères à base de vinyllactame dans les compositions de soins pour la peau et les cheveux, tels que les documents US-A-3 914 403, US-A-3 954 960, US-A-4 039 734, US-A-4 057 533, US-A-4 210 161, US-A-4 223 009, US-A-4 586 518, US-A-4 764 363, US-A-4 834 968, US-4 842 850, US-A-4 902 499, US-A-4 906 459, US-A-4 923 694, US-4 963 348, US-A-5 011 895, US-A-5 015 708, US-A-5 126 124, US-A-5 158 762, US-A-5 275 809, US-A-5 502 136, WO-A-91/15186, WO-A-91/15185, EP-A2-412704, EP-A1-0412707 et JP-A-57126409.

Dans ce même document, on indique également que de nombreux brevets décrivent l'utilisation, en particulier, d'une N-vinyllactame dans le domaine de la cosmétique et des produits pharmaceutiques, en particulier dans les aérosols pour les cheveux. Ces brevets sont les documents US-A-3 910 862, US-A-4 923 694, US-A-5 045 617, US-A-5 321 110, US-A-5 492 988 et US-A-5 637 296.

Ainsi, le document US-A-3 954 960 a trait à des compositions cosmétiques et capillaires qui contiennent en tant que résine filmogène un copolymère quaternisé de vinylpyrrolidone et d'un monomère vinylique copolymérisable à savoir un méth(acrylate) de dialkylaminoalkyle.

Le brevet US-A-3 914 403, quant à lui, est relatif à des compositions capillaires qui contiennent en tant que résine filmogène : un homo ou copolymère de N-vinylpyrrolidone en mélange avec un copolymère quaternisé à base de vinylpyrrolidone et de monomère vinylique copolymérisable avec celle-ci à savoir un méth(acrylate) de dialkylaminoalkyle.

Le document US-A-5 502 136 concerne un procédé de préparation de copolymères de vinylpyrrolidone et d'acétate de vinyle par polymérisation radicalaire.

Le document WO-A-00/68282 a trait à des terpolymères à base de vinylpyrrolidone (VP), de diméthylaminopropyl méthacrylamide (DMAPMA), et du dérivé quaternisé par une chaîne alkyle en C₈ à C₂₄ du DMAPMA, et aux compositions capillaires et cosmétiques les comprenant.

On note que dans ces copolymères des chaînes grasses sont insérées afin de diminuer le collant et d'augmenter la résistance à l'humidité, mais la gamme des propriétés des polymères obtenus et le choix des monomères utilisables pour obtenir des polymères véhiculés dans l'eau se trouvent ainsi limités. Par ailleurs, aucune valeur du collant « tack » de ces copolymères n'est mentionnée.

Le document de Matyjasezwski et al., Prepr. 38(1), 1997, p. 695 décrit des copolymères à squelette N-vinylpyrrolidone et greffons PDMS pour des hydrogels.

Le brevet WO 97/18247 du même K. Matyjaszewski décrit, à la page 103, un exemple d'homopolymère de vinylpyrrolidone.

Le document FR-A-2 327 761 est relatif à des compositions cosmétiques comprenant un polymère résultant de la polymérisation, en présence de cérium, d'un monomère insaturé sur un polyvinylpyrrolidone diol. Un polymère PVP-poly(méthacrylate de lauryle) est mentionné. L'utilisation d'un copolymère vinylpyrrolidone/acrylate de glucosamine est également décrite. Les polymères de ce document sont des polymères très particuliers, généralement à structure branchée.

Ces polymères apportent brillance et tenue dans le temps à la coiffure, mais ils confèrent aussi une certaine rigidité à la coiffure, causant un aspect non naturel.

De plus, ces polymères ne sont pas véhiculés dans l'eau, mais dans les alcools, ou bien dans l'eau en présence de tensioactifs et nécessitent précisément la présence de tensioactifs dans la composition pour favoriser leur élimination au shampooing.

Il existe donc un besoin pour un polymère, qui, lorsqu'il est inclus dans une composition, en particulier une composition cosmétique, fasse en sorte que cette composition ne présente pas les inconvénients, défauts, limitations et désavantages des compositions de l'art antérieur.

Il existe, en particulier, un besoin pour un polymère et une composition le contenant, qui présente une combinaison optimale des propriétés de rigidité, de « collant » notamment au toucher, et d'élimination au shampooing.

Ainsi, une composition capillaire comportant le polymère doit permettre d'obtenir plus de tenue, tout en conservant un effet naturel. Le polymère doit, en outre, dans de telles compositions montrer de bonnes propriétés de coiffage "styling" et, lors du démêlage, ne pas poudrer, c'est-à-dire former des résidus visibles « flaking ». Par ailleurs, le polymère doit être compatible avec les gaz propulseurs aérosols.

Enfin, le polymère, dans de telles compositions capillaires, doit pouvoir être éliminé facilement sous l'action d'un shampooing et, dans tous les cas, présenter une capacité d'élimination améliorée par rapport aux polymères de l'art antérieur.

Dans le cas du maquillage des ongles, l'obtention d'un film brillant est souhaité, ce film devant en plus résister aux agressions mécaniques. Le polymère contenu dans la formule doit donc être capable de résister de manière excellente à l'abrasion mécanique.

Dans le cas d'un traitement de la peau, le maquillage, mis en oeuvre, et qui inclut le polymère, doit adhérer à la peau, sans tirer celle-ci tout en étant confortable (ne pas provoquer des tiraillements).

Dans tous les cas et quelle que soit la composition dans laquelle se trouve utilisé le polymère, il est nécessaire que ce dernier donne un produit non collant au toucher, en particulier en fonction de l'humidité, et notamment dans des conditions d'humidité élevées (à savoir généralement pour des humidités relatives (HR) de 50 à 100 %).

En d'autres termes, il existe un besoin pour un polymère filmogène ne possédant pas, ou peu, de collant ou « tack », et, de toutes façons un collant ou tack, avec l'humidité, diminué par rapport aux polymères filmogènes de l'art antérieur, et qui, en outre, présente une élimination au shampooing améliorée.

Le but de la présente invention est de fournir un polymère qui réponde, entre autres, aux besoins, critères et exigences cités plus haut et qui résolve les problèmes des polymères de l'art antérieur.

Ce but et d'autres encore sont atteints, conformément à la présente invention, par un copolymère éthylénique, séquencé, linéaire comprenant :
- au moins une séquence A susceptible d'être préparée à partir de monomères, constitués de 52 à 99% en poids d'un monomère éthylénique à cycle lactame répondant à la formule (I) suivante : 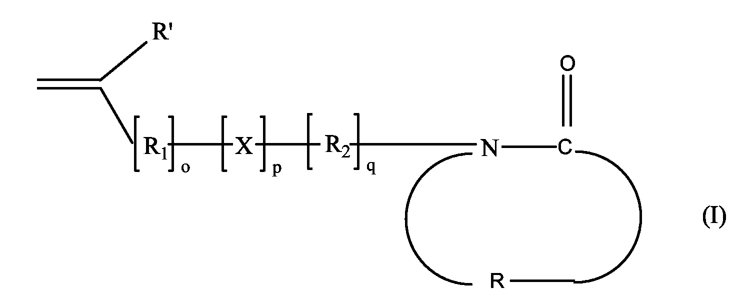 dans laquelle :
   - R représente un groupe -(CH₂)ₙ-, où un ou plusieurs des atomes de carbone sont éventuellement remplacés par un atome d'azote ou un atome d'oxygène, où n est un nombre entier de 3 à 12, et où un ou plusieurs des atomes de carbone sont éventuellement substitués par un ou plusieurs groupes alkyle en C₁ à C₆ ;
   - R' représente H ou un groupe méthyle ;
   - R₁ et R₂, qui peuvent être identiques ou différents représentent un groupe alkylène ou aralkylène, linéaire, ramifié, ou cyclique, de 1 à 22 C, dans lequel un ou plusieurs des atomes de carbone sont éventuellement remplacés par un atome d'oxygène ou d'azote ;
   - X est choisi parmi -OCO-, -NHCO-, -COO-, -O- ;
   - o, p, q, représentent chacun indépendamment les uns des autres 0 ou 1 ;
   et de 1 à 48% en poids d'au moins un monomère hydrophile ionique ;
- et au moins une séquence B susceptible d'être préparée à partir de monomères ne comportant pas de monomère éthylénique à cycle lactame de formule (I) ou comportant une proportion minoritaire de celui-ci.

La teneur (52 à 99% en poids) en monomère éthylénique à cycle lactame et la teneur en monomère hydrophile ionique (1 à 48% en poids) sont données par rapport à poids total des monomères à partir desquels est susceptible d'être préparée la séquence A.

En d'autres termes, la teneur en motifs issus d'un monomère éthylénique à cycle lactame de la séquence A est de 52 à 99% en poids et la teneur en motifs issus d'un monomère hydrophile ionique de la séquence A est de 1 à 48% en poids.

Ces teneurs sont données par rapport au poids total de la séquence A.

De préférence dans la formule (I), o est 0, p est 1, q est 1, R₂ représente -CH₂CH₂-, X représente COO ou CONH, et R est (CH₂)₃ ou (CH₂)₅ ou (CH₂CH₂NH).

Le monomère de formule (I) sera donc de préférence un méthacrylate ou acrylate de pyrrilidonoéthyle, ou un acrylate ou méthacrylate d'uréidoéthyle.

De préférence, la séquence A est susceptible d'être préparée à partir de monomères, constitué(s) de 52 à 99% en poids d'un monomère éthylénique à cycle lactame qui est un vinyllactame, répondant à la formule (II) suivante : 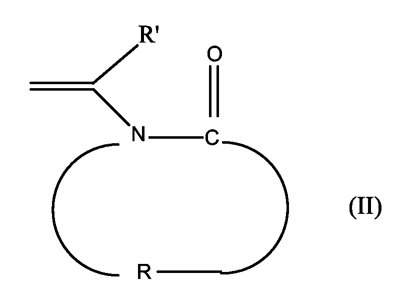 dans laquelle R et R' ont la signification déjà donnée plus haut.

De préférence, dans les formules (I) et (II) ci-dessus, R représente -(CH₂)n-, avec n est un nombre entier de 3 à 5, ou bien R est -CH₂-CH₂-NH-.

Avantageusement, le N-vinyllactame de formule (II) est le N-vinylpyrrolidone (n=3), le N-vinylpipéridinone (valérolactame) (n=4), le N-vinylcaprolactame (n=5), la N-vinylimidazolidinone dans laquelle R est le groupe -CH₂-CH₂-NH-, le N-vinyl-5-méthyl-2-pyrrolidone, le N-vinyl-5-éthyl-2-pyrrolidone, le N-vinyl-6-méthyl-2-pipéridone, le N-vinyl-6-éthyl-2-pipéridone, le N-vinyl-7-méthyl-2-caprolactame, ou le N-vinyl-7-éthyl-2-caprolactame.

Les N-vinyllactames préférés sont la N-vinylpyrrolidone, la N-vinylcaprolactame et la vinylimidazolidinone.

L'invention a également pour objet les compositions cosmétiques ou pharmaceutiques comprenant lesdits copolymères éthyléniques, séquencés, linéaires.

Lorsqu'ils sont incorporés dans de telles compositions, les copolymères présentant la structure spécifique, selon l'invention, permettent d'obtenir des propriétés, ou plutôt une combinaison de propriétés extrêmement intéressantes, qu'il n'était pas possible d'obtenir avec les polymères de l'art antérieur.

De manière générale, les copolymères selon l'invention présentent, du fait de leur structure particulière une hygroscopie réduite par rapport aux copolymères de l'art antérieur présentant des motifs vinyllactames.

Les copolymères de l'invention ont une combinaison optimale de rigidité et de caractère non collant et ils conduisent ainsi à des compositions ou systèmes ayant notamment des résistances mécaniques, des résistances à l'usure et des tenues dans le temps améliorées, et une fragilité réduite, tout en n'étant pas collants.

Les copolymères selon l'invention présentent, en outre, la propriété particulière de pouvoir être éliminés facilement par un shampooing, et, en tous cas, plus facilement que les polymères de l'art antérieur.

Les copolymères selon l'invention qui sont, de préférence, des polymères filmogènes peuvent donc être définis comme des polymères ne présentant pas ou peu de collant ou « tack ».

Ainsi, lorsque les copolymères, selon l'invention, sont utilisés dans des compositions pour le traitement de la chevelure, telles que des laques, ils apportent plus de tenue dans le temps. Ils sont moins fragiles qu'une laque classique et simultanément ils ne sont pas collants. Le phénomène de poudrage sur les cheveux, observé lors du démêlage, avec les compositions de l'art antérieur est évité.

De plus, ces compositions peuvent être éliminées facilement par un shampooing et, en tous cas, plus facilement que les compositions comprenant les polymères de l'art antérieur.

Dans le cas des vernis à ongles, le vernis comprenant le copolymère selon l'invention, a une résistance à l'usure supérieure et ne colle pas, tout en adhérant sur l'ongle. De ce fait, la perte de brillance, c'est-à-dire la matification du film par le marquage mécanique ou par le marquage par les poussières qui se produit avec des films collants de l'art antérieur ne se produit pas avec les vernis et films comprenant le copolymère selon l'invention. En effet, l'absence de collant, « tack », de ces derniers fait qu'ils ne sont pas marqués mécaniquement et qu'ils ne retiennent pas les poussières et qu'ils ne subissent donc pas de modification ou perte de brillance.

Les vernis, comprenant le copolymère selon l'invention, grâce à la structure particulière de celui-ci, donnent des films qui ne s'écaillent pas et qui, de manière étonnante, ne sont pas collants.

Dans les produits de maquillage, comme les rouges à lèvres ou les fonds de teint, le maquillage présente une bonne tenue sur les lèvres ou la peau, sans laisser de sensation de collant.

L'invention concerne également un procédé cosmétique de maquillage ou de soin des matières kératiniques comprenant l'application sur les matières kératiniques d'une composition selon l'invention.

L'invention concerne donc, en outre, l'utilisation des copolymères selon l'invention pour améliorer la tenue de la coiffure, sans collant, notamment au toucher, d'une laque pour cheveux ; l'utilisation des copolymères, selon l'invention, pour améliorer l'élimination par un shampooing, d'une composition capillaire, telle qu'une laque ; l'utilisation des copolymères selon l'invention pour améliorer l'adhérence et la résistance à l'usure, sans collant, notamment au toucher, d'un vernis à ongles; l'utilisation des copolymères selon l'invention pour optimiser l'adhérence sur la peau, et le confort d'une composition de maquillage ; l'utilisation des copolymères selon l'invention pour diminuer le collant, notamment au toucher, d'une composition cosmétique, en particulier dans des conditions d'humidité élevée, par exemple pour des humidités relatives (HR) de 50 à 100 % ; l'utilisation des copolymères selon l'invention dans une composition cosmétique telle qu'une composition de maquillage pour masquer les rides qui donne à la peau un aspect lissé, sans tiraillement.

Les copolymères, selon l'invention, apportent donc une solution aux problèmes posés par les polymères de l'art antérieur.

Les propriétés avantageuses, inattendues, des copolymères spécifiques de l'invention, qui sont fondamentalement des copolymères linéaires, proviennent notamment, voire essentiellement, de la nature spécifique des séquences qui les constituent et, en particulier, de la séquence A, constituée de 52 à 99% en poids d'un monomère éthylénique à cycle lactame, tel qu'un vinyllactame.

De manière étonnante, les copolymères selon l'invention, grâce, semble-t-il, à la présence dans la séquence A constituée de 52 à 99% en poids d'un monomère éthylénique à cycle lactame, tel qu'un vinyllactame, d'un ou plusieurs autres monomères (1 à 48% en poids), qui sont spécifiquement choisis parmi les monomères hydrophiles ioniques, sont beaucoup plus facilement éliminés par un shampooing que les polymères à base de vinyllactames de l'art antérieur, tout en ayant une hygroscopie réduite par rapport aux polymères à base de vinyllactame de l'art antérieur.

Notamment, les copolymères de l'invention sont beaucoup plus facilement éliminés par un shampooing et ont une hygroscopie réduite lorsqu'on les compare aux copolymères non ioniques de vinyllactame de l'art antérieur.

De même, les copolymères de l'invention sont, de manière surprenante, plus facilement éliminés par un shampooing et ont une hygroscopie réduite (attestée par des valeurs de tack plus faibles en humidité forte, par exemple pour des HR de 50 à 100 %), lorsqu'on les compare aux copolymères de l'art antérieur présentant une structure séquencée, formée de séquences constituées d'homopolymères de vinyllactame (par exemple, de polyvinylpyrrolidone PVP) et de séquences préparées à partir de monomères non hydrophiles, tels que le méthacrylate de méthyle.

Il s'est en effet avéré, de manière étonnante, que l'incorporation d'un monomère hydrophile, plus particulièrement d'un monomère hydrophile ionique, au sein même de la séquence de vinyllactame, qui se présente donc selon l'invention spécifiquement comme un copolymère (que ce copolymère soit alterné, à gradient, ou statistique), du monomère éthylénique à cycle lactame, tel qu'un vinyllactame, et du monomère hydrophile ionique conduisait à une amélioration significative de l'élimination par un shampooing par rapport à un copolymère analogue, mais dans lequel les séquences de polyvinyllactame sont constitués par des homopolymères de vinyllactames et ne comportent donc pas de monomères hydrophiles ioniques. Cet avantage, cette amélioration sont inhérents au fait que la séquence soit un copolymère et sont présents quel que soit le type de copolymère statistique, alterné ou à gradient.

De la même manière, l'élimination sous l'action d'un shampooing des copolymères de l'invention est très fortement améliorée par rapport à des copolymères de l'art antérieur préparés à partir de vinyllactames et d'un monomère hydrophile non ionique. De manière étonnante, le choix spécifique d'un monomère hydrophile, ionique, assure une excellente élimination au shampooing, alors que les copolymères préparés à partir de vinyllactames et d'un monomère hydrophile non ionique, tels que ceux décrits dans le document FR-A-2 327 761, ne sont éliminés que difficilement, voire pas du tout, et les compositions contenant ces copolymères avec un monomère non ionique nécessitent l'incorporation d'un tensioactif ionique dans la composition pour faciliter ou assurer l'élimination du shampooing.

Parmi les monomères hydrophiles ioniques, il s'est avéré que l'effet d'amélioration de l'élimination au shampooing était encore accru avec les monomères hydrophiles anioniques.

Le copolymère selon l'invention est caractérisé, en outre, par le fait qu'il comporte, outre la séquence A, préparée à partir d'un monomère éthylénique à cycle lactame, tel qu'un vinyllactame (52 à 99% en poids), une autre séquence B, exempte de monomère éthylénique à cycle lactame, tel qu'un vinyllactame ou dans laquelle le monomère éthylénique à cycle lactame, tel qu'un vinyllactame est en minorité. Les copolymères selon l'invention se différencient donc de certains copolymères de l'art antérieur qui sont des copolymères statistiques de vinyllactame, par exemple de vinylpyrrolidone et d'un autre monomère, par exemple hydrophobe, et qui ne comportent donc pas de séquence B selon l'invention.

De tels copolymères statistiques de l'art antérieur qui sont, par exemple décrits dans le document WO-A-00 68282, cité plus haut, de par la présence de motifs hydrophobes, sont difficilement éliminables au shampoing. Au contraire, les copolymères selon l'invention peuvent présenter des structures variées, du fait de leur structure séquencée avec une séquence B et donc conduire à des propriétés adaptées à l'application.

La séquence B peut être choisie à volonté pour communiquer au copolymère selon l'invention toutes les propriétés souhaitables voulues - outre le caractère non collant - qui correspondent à des applications spécifiques. Par exemple, la séquence B pourra être choisie de manière à contrôler la flexibilité du copolymère et obtenir de manière surprenante des copolymères qui ne sont pas fragiles, friables, et qui donnent des films sans écailles, tout en n'étant pas collants, notamment au toucher, et en étant très facilement éliminables par un shampooing.

Si l'on fait en sorte que la séquence B soit hydrophile, alors l'excellente capacité à être éliminée par un shampooing des copolymères selon l'invention se trouvera encore accrue.

Rien ne laissait supposer dans l'art antérieur qu'en mettant en oeuvre un copolymère spécifiquement linéaire, et en imposant :
- qu'au moins une séquence (A) de ce copolymère soit constituée de 52 à 99% en poids de monomère éthylénique à cycle lactame, tel qu'un vinyllactame ;
- que le ou les autres monomères de cette séquence (A) soient spécifiquement hydrophiles et plus particulièrement hydrophiles ioniques ; et, enfin,
- qu'au moins une autre séquence (B), ait une structure différente de la séquence (A) et soit exempte de, ou constituée en minorité, de monomère éthylénique à cycle lactame, tel qu'un N-vinyllactame,
on pourrait parvenir, selon l'invention, tout d'abord à une amélioration de l'élimination du copolymère ou d'une composition le contenant par un shampooing, ensuite à une diminution sans précédents, du caractère collant du copolymère, et enfin à l'obtention, en choisissant la séquence B voulue, d'une combinaison de propriétés excellentes pour ce copolymère.

La structure spécifique du copolymère de l'invention conduit à une optimisation de ses propriétés, conduisant à un parfait équilibre entre, par exemple, les propriétés de résistance mécanique, de non-collant, notamment au toucher, et de facilité d'élimination par un shampooing.

Sans vouloir être lié par aucune théorie, les propriétés avantageuses du copolymère selon l'invention proviendraient du fait que la nature des séquences est spécifiquement choisie de façon à favoriser la séparation des phases entre celles-ci et donc, entre autres, de contrôler de manière optimale la rigidité et l'hygroscopie et donc le collant du copolymère, ainsi que la capacité du copolymère à être éliminé par un shampooing.

Par ailleurs, le fait que le polymère soit linéaire implique une synthèse beaucoup plus facile et contrôlée, ce qui permet de prévoir de façon précise la structure des polymères obtenus, et ainsi d'optimiser les propriétés finales des polymères.

De manière plus précise, les copolymères selon l'invention sont des copolymères séquencés ou blocs. On entend généralement par ces termes que les copolymères sont constitués de séquences ou blocs accrochés les uns aux autres de façon covalente.

En outre, deux séquences successives sont de natures différentes. Par contre, deux séquences A ou B non successives peuvent être de même nature. Chaque séquence A est constituée d'un copolymère de monomère éthylénique à cycle lactame, tel qu'un vinyllactame, et du monomère hydrophile ionique, ce qui génère un copolymère statistique, alterné, ou à gradient.

La séquence B est constituée d'un homopolymère ou d'un copolymère, celui-ci pouvant à son tour être statistique, alterné, ou à gradient.

Le polymère peut comporter en outre une autre séquence C différente des séquences A et B, et éventuellement encore d'autres séquences, par exemple une séquence D différente de A, B et C.

Le polymère selon l'invention pourra donc être choisi parmi les copolymères biséquencés de type AB, les copolymères triséquencés de type ABA, BAB, ABC, ACB avec C différent de A ou B.

Le polymère selon l'invention pourra aussi être choisi parmi les copolymères multiséquencés ayant plus de trois séquences différentes ou non de type (AB)ₙ, (ABA)ₙ, (BAB)ₙ, (ABC)ₙ, (ACB)ₙ avec C différent de A ou B, ou les copolymères multiséquencés ayant plus de trois séquences différentes de type ABCD.

De manière générale, comme on l'a déjà indiqué, la nature des séquences est choisie de manière à favoriser la séparation des phases entre les séquences, puisque celle-ci prédétermine les propriétés.

La nature des séquences et leur nombre sont choisis par l'homme du métier en fonction des propriétés recherchées dans les limites des conditions spécifiées ci-dessus pour les séquences A et B.

Les copolymères de l'invention sont définis comme étant des copolymères éthyléniques. Cela signifie que les monomères dont sont issus les séquences ou blocs constituant le copolymère sont des monomères à double liaison insaturée carbone-carbone de type éthylénique.

En outre, spécifiquement, le copolymère, selon l'invention, est un copolymère linéaire. Cela signifie que l'invention n'entend pas couvrir les copolymères ayant une structure non linéaire, par exemple ramifiée, en étoile, greffée, ou autre. Le caractère linéaire des copolymères de l'invention est important pour communiquer aux compositions le contenant, les propriétés avantageuses décrites plus haut.

Avantageusement, le copolymère est un polymère filmogène, c'est-à-dire qu'il est apte à lui seul, ou en présence d'agent auxiliaire de filmification, à la température allant de 20°C à 30°C, à former un film continu (vu à l'oeil nu) et adhèrent sur un support kératinique.

Selon l'invention, le copolymère comprend au moins une séquence A susceptible d'être préparée à partir de monomères constitués de 52 à 99% en poids d'un monomère éthylénique à cycle lactame, par exemple d'un vinyllactame, de formule (I) et de préférence de formule (II).

La teneur (52 à 99% en poids) en monomère éthylénique à cycle lactame et la teneur en monomère hydrophile ionique (1 à 48% en poids) sont données par rapport à poids total des monomères à partir desquels est susceptible d'être préparée la séquence A.

En d'autres termes, la teneur en motifs issus d'un monomère éthylénique à cycle lactame de la séquence A est de 52 à 99% en poids et la teneur en motifs issus d'un monomère hydrophile ionique de la séquence A est de 1 à 48% en poids.

Ces teneurs sont données par rapport au poids total de la séquence A.

Le pourcentage de monomère éthylénique à cycle lactame, tel qu'un vinyllactame de formule (I) ou (II), dans les monomères à partir desquels la séquence A est susceptible d'être préparée est de 52 à 99 %, de préférence de 55 à 95 % en poids, de préférence encore de 60 à 80% en poids, mieux de 65 à 75% en poids, par exemple de 70 % en poids.

En d'autres termes, la teneur (en poids) en motifs issus d'un monomère éthylénique à cycle lactame de la séquence A est de 52 à 99% en poids. Et généralement, de préférence, la teneur (en poids) en motifs issus d'un monomère éthylénique à cycle lactame de la séquence A est dans les plages de % en poids de 55 à 95% en poids, etc ... définies ci-dessus. Ces teneurs et teneurs préférées sont données par rapport au poids total de la séquence A.

La séquence A constituée de 52 à 99% en poids d'un monomère éthylénique à cycle lactame, par exemple en vinyllactame, peut avoir une température de transition vitreuse (Tg) globale de la séquence quelconque, mais elle a généralement une température de transition vitreuse globale « haute ».

Par "haute", on entend généralement que cette Tg peut être de 0 à 250°C, de préférence de 0 à 220°C, et de préférence encore de 5 à 200°C.

La séquence A ne comprend que des monomères hydrophiles.

Rappelons qu'une séquence hydrophile peut être définie comme une séquence hydrosoluble ou hydrodispersible.

La séquence A est donc, de préférence, une séquence hydrophile.

Elle peut cependant présenter un caractère hydrophobe en fonction du neutralisant utilisé pour neutraliser la fonction ionique.

Le terme hydrophile est défini plus haut.

Le monomère éthylénique à cycle lactame, tel qu'un N-vinyllactame, peut être choisi parmi les N-vinyllactames et les dérivés de ceux-ci qui peuvent avoir, par exemple, un ou plusieurs substituants alkyle en C₁ à C₆, tels que méthyle, éthyle n-propyle, isopropyle, n-butyle, sec-butyle.

Avantageusement, ledit monomère éthylénique à cycle lactame, tel qu'un N-vinyllactame, de formule (I) est le pyrrilidinoéthylacrylate, ou le pyrrilidinoéthylméthacrylate.

Avantageusement, le N-vinyllactame de formule (II) est le N-vinylpyrrolidone (n=3), le N-vinylpipéridinone (valérolactame) (n=4), le N-vinylcaprolactame (n=5), la N-vinylimidazolidinone dans laquelle R est le groupe -CH₂-CH₂-NH-, le N-vinyl-5-méthyl-2-pyrrolidone, le N-vinyl-5-éthyl-2-pyrrolidone, le N-vinyl-6-méthyl-2-pipéridone, le N-vinyl-6-éthyl-2-pipéridone, le N-vinyl-7-méthyl-2-caprolactame, ou le N-vinyl-7-éthyl-2-caprolactame.

Les N-vinyllactames préférés sont la N-vinylpyrrolidone, la N-vinylcaprolactame et la vinylimidazolidinone.

Selon l'invention, la séquence A, constituée de 52 à 99% en poids d'un monomère éthylénique à cycle lactame, tel qu'un N-vinyllactame, est un copolymère, les monomères, à partir desquels elle est susceptible d'être préparée, sont donc constituées, outre le monomère éthylénique à cycle lactame, tel qu'un vinyllactame, de formule (I), par un ou plusieurs autres monomères, "additionnels" identiques ou différents, et ce ou ces monomère(s), sont, selon l'invention, des monomères hydrophiles ioniques.

Les monomères à partir desquels est susceptible d'être préparée la séquence A, sont constitués par une proportion de monomère(s) hydrophile(s) ionique(s) de 1 à 48% en poids par rapport au poids total des monomères à partir desquels est susceptible d'être préparée la séquence A. De préférence, le pourcentage de monomère hydrophile ionique dans les monomères à partir desquels est susceptible d'être préparée la séquence A est de 5 à 45%, de préférence encore de 20 à 40%, mieux de 25 à 35%, par exemple de 30%.

En d'autres termes, la teneur (en poids) en motifs issus d'au moins un monomère hydrophile ionique de la séquence A est de 1 à 48% en poids. Et généralement, de préférence, la teneur (en poids) en motifs issus d'un monomère hydrophile ionique de la séquence A se situe dans les plages préférées définies ci-dessus. Ces teneurs et teneurs préférées sont données par rapport au poids total de la séquence A.

Ce ou ces monomères de la séquence A, autres que le monomère éthylénique à cycle lactame, tel que le vinyllactame sont des monomères éthyléniques copolymérisables avec le monomère dérivé lactame quelque soit leur coefficient de réactivité.

La séquence A, constituée de 52 à 99% en poids d'un monomère éthylénique à cycle lactame, tel qu'un vinyllactame, représente généralement 50 % en poids ou plus du poids total du copolymère de l'invention, de préférence de 50 à 99 %, de préférence encore de 55 à 95 %, mieux de 60 à 90 %.

La ou les séquences B autres que la séquence A est(sont) susceptible(s) d'être préparée(s) à partir d'un ou de plusieurs monomères éthyléniques choisis généralement parmi : les monomères allyliques, les acrylates, les méthacrylates, les acrylamides, les méthacrylamides, les monomères vinyliques, et leurs mélanges, et éventuellement des monomères éthyléniques à cycle lactame, par exemple des monomères de vinyllactame, de formule (I) ou (II), la proportion en poids desdits monomères éthyléniques à cycle lactame, par exemple des vinyllactames, (I) ou (II), étant minoritaire, inférieure à 50% en poids, de préférence inférieure ou égale à 45 % en poids et, de préférence encore, inférieure ou égale à 40 % en poids, mieux inférieure ou égale à 30 % en poids.

La ou les séquences B peut(peuvent) être hydrophobe(s) ou hydrophile(s), de préférence, une ou plusieurs parmi les séquences B, autres que la séquence A, sont hydrophiles, ce qui accroît encore la facilité d'élimination par un shampooing.

De préférence, au moins une des séquences B a une température de transition vitreuse Tg, basse, à savoir, de préférence, inférieure ou égale à 50°C.

Avantageusement, la masse moléculaire (moyenne en nombre) de chaque bloc ou séquence, qu'il s'agisse de la séquence A ou de la séquence B, est de 2000 à 1 000 000, de préférence elle est de 2000 à 800 000, de préférence encore de 2 000 à 500 000.

La masse moléculaire moyenne en nombre du copolymère global (par exemple A-b-B) est généralement de 4 000 à 1 000 000, de préférence de 4 000 à 800 000, de préférence encore de 4 000 à 500 000.

Avantageusement, la proportion de la séquence B est comprise entre 1 et 50 % en poids du poids total du copolymère, de préférence entre 5 et 45 % et, de préférence encore entre 10 et 40 %.

On va maintenant décrire, de manière plus précise, le copolymère selon l'invention.

La température de transition vitreuse Tg étant un paramètre important pour définir les séquences du copolymère de l'invention, notamment la séquence B du copolymère de l'invention et, par voie de conséquence, le copolymère de l'invention, il est important d'indiquer que les températures de transition vitreuse des séquences des copolymères utilisés dans la présente invention sont mesurées par analyse enthalpique différentielle (DSC, « Differential Scanning Calorimetry », en anglais) pour le polymère sec, à une vitesse de chauffe de 10°C/minute.

Chaque séquence B du copolymère, selon l'invention, est issue d'un type de monomère ou de plusieurs types de monomères différents.

Cela signifie que chaque séquence B peut être constituée d'un homopolymère ou d'un copolymère ; ce copolymère constituant la séquence pouvant être à son tour statistique, alterné ou à gradient.

Chaque séquence A du copolymère selon l'invention est constitué par un copolymère, qui peut être alterné, statistique ou à gradient ; ce copolymère comprenant, bien sûr, une proportion de 52 à 99% en poids de monomère éthylénique à cycle lactame, tel qu'un vinyllactame.

Nous allons décrire maintenant en détail chacune des séquences A et B constituant le copolymère selon l'invention.

Selon l'invention, la séquence A, constituée de 52 à 99% en poids de monomère éthylénique à cycle lactame, tel qu'un vinyllactame, est constituée en outre de 1 à 48% en poids, d'au moins un monomère hydrophile ionique.

Par monomère hydrophile, on entend que l'homopolymère préparé à partir de ce monomère est hydrosoluble ou hydrodispersible, ou qu'il peut être rendu hydrosoluble ou hydrodispersible par suite d'un traitement telle qu'une hydrolyse, par exemple.

Parmi les monomomères hydrophiles ioniques, on compte ainsi les monomères anioniques, cationiques, et les bétaïnes, tels que décrits ci-dessous.

On compte aussi parmi ces monomères les monomères pouvant être rendus hydrophiles (ioniques) par suite d'une hydrolyse, il s'agit en particulier des monomères de type esters (méth)acryliques hydrolysables en acide.

L'homopolymère est hydrosoluble, s'il est soluble dans l'eau, à raison d'au moins 5 % en poids, à 25°C.

L'homopolymère est hydrodispersible, s'il forme à une concentration de 5 %, à 25°C, une suspension stable de fines particules, généralement sphériques. La taille moyenne des particules constituant ladite dispersion est inférieure à 1 µm et, plus généralement, varie entre 5 et 400 nm, de préférence de 10 à 250 nm. Ces tailles de particules sont mesurées par diffusion de lumière.

Le ou les monomère(s) hydrophiles ioniques minoritaires, autres que le monomère éthylénique à cycle lactame, tel qu'un vinyllactame, à partir desquels est préparé la séquence A, sont, de préférence, choisis parmi les monomères cationiques et les monomères anioniques.
- Des exemples de monomères cationiques sont :
   - la 2-vinylpyridine (Tg : 104°C) ;
   - la 4-vinylpyridine (Tg : 142°C) ;
   - le (méth)acrylate de diméthylaminoéthyle ;
   - le (méth)acrylate de diéthylaminoéthyle ;
   - le diméthylaminopropyl(méth)acrylamide ; et
   les formes salifiées ou quaternisées de ceux-ci, qu'il s'agisse de sels d'acides minéraux, tels que l'acide sulfurique ou l'acide chlorhydrique, ou de sels d'acides organiques.
   Ces acides organiques peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyles.
   Un exemple d'acide à groupe alkyle est l'acide acétique CH₃COOH.
   Un exemple de polyacide est l'acide téréphtalique.
   Des exemples d'hydroxyacides sont l'acide citrique et l'acide tartrique.
- Des exemples de monomères anioniques sont :
   - l'acide acrylique, l'acide méthacrylique, l'acide crotonique, l'anhydride maléique, l'acide itaconique, l'acide fumarique, l'acide maléique, l'acide vinylbenzoïque ;
   - l'acide styrènesulfonique, l'acide 2-acrylamido 2-methylpropanesulfonique, l'acide vinylsulfonique,
   - l'acide vinylphosphonique, le méthacrylate de sulfopropyle, et les sels de ceux-ci.

   Le neutralisant peut être une base minérale, telle que LiOH, NaOH, KOH, Ca(OH)₂, NH₄OH ; une base organique. Il peut s'agir d'une alkylamine primaire, secondaire ou tertiaire, telle que la triéthylamine, ou la butylamine. Cette alkylamine,primaire, secondaire ou tertiaire , peut comporter des azotes et/ou oxygènes, et donc comporter par exemple une fonction alcool, par exemple l'amino-2-méthyl-2-propanol,ou la triéthanolamine.
- Les monomères pouvant être hydrophiles par suite d'une hydrolyse sont en particulier les monomères de type esters (méth)acryliques hydrolysables en acide tels que les (méth)acrylate d'éthyle, de teriobutyle, de benzyle.

On procèdera à l'hydrolyse, une fois le polymère synthétisé, selon des conditions acides (tels que l'acide sulfurique, acide chlorhydrique ou acide trifluoroacétique) ou basiques (en présence d'hydroxydes de métaux alcalinoterreux tels que la soude ou la potasse, d'alcoolates de métaux alcalins tels que le t-butylate de potassium, ou d'amines telles que l'ammoniaque). L'hydrolyse a généralement lieu entre 5 et 100°C, de préférence entre 15 et 80°C.

Le polymère sera ensuite purifié par précipitations répétées.

Les monomères anioniques sont préférés car ils conduisent à une amélioration encore supérieure de l'élimination par un shampooing.

Les monomères minoritaires hydrophiles ioniques préférés sont :
- l'acide acrylique, l'acide méthacrylique, l'acide crotonique, l'anhydride maléique, l'acide vinylbenzoïque, l'acide styrènesulfonique, l'acide 2-acrylamido-2-méthylpropanesulfonique, l'acide vinylsulfonique,
- l'acrylate de tertiobutyle ou l'acrylate d'éthyle suivi de l'hydrolyse.

Le copolymère selon l'invention comprend également une ou plusieurs autres séquences B qui est(sont) susceptible(s) d'être préparée(s) à partir de monomères ne comportant pas de monomère éthylénique à cycle lactame, tel qu'un vinyllactame, de formule (I) ou comportant une proportion minoritaire de celui-ci. Il n'existe aucune limitation quant à la nature des monomères qui sont susceptibles de donner la séquence B, à l'exception, bien sûr, de la limitation quant à la quantité de monomère éthylénique à cycle lactame, tel qu'un vinyllactame susceptible d'être utilisée pour la préparation de cette séquence B.

Ces monomères peuvent être hydrophobes, non hydrophiles, mais ils peuvent aussi être hydrophiles. Les termes « hydrophile » et a contrario « non hydrophile » ont déjà été définis plus haut.

Le ou les monomères à partir desquels la séquence ou les séquences B est(sont) susceptible(s) d'être préparée(s) peuvent être choisis parmi une grande variété de monomère(s), les Tg de ces monomères peuvent être quelconques, et ces monomères peuvent en outre être hydrophiles ou hydrophobes.

Le ou les monomères à partir desquels la séquence ou les séquences B est(sont) susceptible(s) d'être préparée(s) peuvent donc, par exemple, être choisi(s) parmi les monomères décrits ci-après.
- Les hydrocarbures éthyléniques de 2 à 10 C, tels que l'éthylène, l'isoprène, ou le butadiène ;
- Les acrylates de formule CH₂ = CHCOOR₃ ;
- Les méthacrylates de formule : 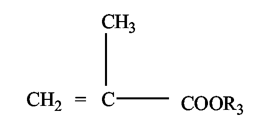 dans lesquelles R₃ représente :
   - un groupe alkyle linéaire, ou ramifié, de 1 à 18 atomes de carbone, dans lequel se trouve(nt) éventuellement intercalé(s) un ou plusieurs hétéroatomes choisis parmi 0, N, S et P,
   ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆ ou un groupe phényle,
   des exemples de ces groupes alkyle sont méthyle, éthyle, propyle, butyle, isobutyle, tertiobutyle, éthylhexyle, octyle, lauryle et stéaryle,
   des exemples de ces groupes dérivés d'alkyle sont les groupes hydroxyalkyle en C₁₋₄ tels que 2-hydroxyéthyle et 2-hydroxypropyle, et les groupes alcoxy (C₁₋₄) alkyle (C₁₋₄) tel que méthoxyéthyle, éthoxyéthyle et méthoxypropyle,
   - un groupe cycloalkyle en C₃ à C₁₂, tel que le groupe isobornyle,
   - un groupe aryle en C₃ à C₂₀ tel que le groupe phényle,
   - un groupe aralkyle en C₄ à C₃₀ (groupe alkyle en C₁ à C₈) tel que 2- phényl-éthyle ou benzyle,
   - un groupe hétérocyclique de 4 à 12 chaînons contenant un ou plusieurs hétéroatomes choisis parmi O, N, et S, le cycle étant aromatique ou non,
   - un groupe hétérocyclylalkyle (alkyle de 1 à 4 C), tel que furfurylméthyle ou tétrahydrofurfurylméthyle,
   lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆, ou un groupe phényle,
   - des exemples de groupes R₃ sont les groupes méthyle, éthyle, propyle , isobutyle, n-butyle, tertiobutyle isobutyle, héxyle, éthylhéxyle, octyle, lauryle, isooctyle, isodécyle, dodécyle, cyclohexyle, t-butylcyclohexyle, t-butylbenzyle, isobornyle, phényle, furfurylméthyle, tétrahydrofurfurylmethyle, 2-hydroxyéthyle, 2-hydroxypropyle, 2-hydroxybutyle, méthoxyéthyle, éthoxyéthyle, méthoxyéthyle, méthoxypropyle, éthyl-2-perfluorohexyle,
   - un autre exemple de groupe R₃ pour les acrylates sont les groupes R₃ = -(OC₂H₄)ₘ-OR'', avec m = 5 à 150 et , R" = H ou alkyle de C₁ à C₃₀, par exemple -POE- méthoxy ; POE-béhényle ;
- Les (méth)acrylamides de formule : 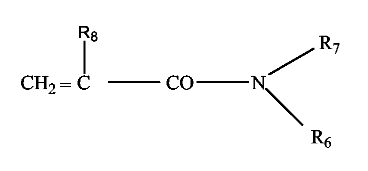 où
   R₈ désigne H ou méthyle ;
   et R₇ et R₆ identiques ou différents représentent chacun un atome d'hydrogène ou un groupe alkyle de 1 à 18 atomes de carbone linéaire ou ramifié, dans lequel se trouve(nt), éventuellement intercalé(s) un ou plusieurs hétéroatomes choisis parmi 0, N, S et P, ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents, représentent un groupe alkyle en C₁ à C₆, ou un groupe phényle,
   des exemples de ces groupes sont les groupes méthyle, éthyle, n-butyle, t-butyle, isopropyle, isohexyle, isooctyle, isononyle, et hydroxyalkyle en C₁₋₄ tels que 2-hydroxyéthyle,
   - un groupe cycloalkyle en C₃ à C₁₂, tel que le groupe isobornyle,
   - un groupe aryle en C₃ à C₂₀ tel que phényle,
   - un groupe aralkyle en C₄ à C₃₀ (groupe alkyle en C₁ à C₈) tel que 2- phényl éthyle ou benzyle,
   - un groupe hétérocyclique de 4 à 12 chaînons contenant un ou plusieurs hétéroatomes choisis parmi O, N, et S, le cycle étant aromatique ou non,
   - un groupe hétérocyclylalkyle (alkyle de 1 à 4 C), tel que furfurylméthyle ou tétrahydrofurfurylméthyle,
   lesdits groupes cycloalkyle, aryle, aralkyle, hétérocyclique ou hétérocyclylalkyle pouvant être éventuellement substitué(s) par un ou plusieurs substituants choisi(s) parmi les groupes hydroxyle, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si (R₄R₅), où R₄ et R₅ identiques ou différents représentent un groupe alkyle en C₁ à C₆, ou un groupe phényle.
   Des exemples de monomères (méth)acrylamide sont le(méth)acrylamide, le N-éthyl(méth)acrylamide, le N-butylacrylamide, le N-t-butylacrylamide, le N-isopropylacrylamide, le N,N-diméthyl(méth)acrylamide, le N,N-dibutylacrylamide, le N-octylacrylamide, le N-dodécylacrylamide, l'undécylacrylamide et le N(2-hydroxylpropyl méthacrylamide) ;
- Les composés allyliques de formule :
   CH₂ = CH-CH₂-R₉ ou CH₂ = C(CH₃)-CH₂-R₉ ;
- Les composés vinyliques de formule :
   CH₂ = CH-R₉,
   où R₉ est un groupe
   - hydroxyle,
   - Cl,
   - NH₂,
   - OR₁₀, où R₁₀ représente un groupe phényle ou un groupe alkyle en C₁ à C₁₂ (le monomère est un éther de vinyle ou d'allyle),
   - Acétamide : NHCOCH₃,
   - OCOR₁₁, où R₁₁ représente :
      - un groupe alkyle de 2 à 12 C linéaire ou ramifié (le monomère est un ester de vinyle ou d'allyle) ;
      - un groupe cycloalkyle en C₃ à C₁₂ tel que isobornyle, cyclohexyle,
      - un groupe aryle en C₃ à C₂₀ tel que phényle,
      - un groupe aralkyle en C₄ à C₃₀ (groupe alkyle en C₁ à C₈) tel que 2-phényléthyle ; benzyle,
      - un groupe hétérocyclique de 4 à 12 chaînons contenant un ou plusieurs hétéroatomes choisis parmi O, N, et S, le cycle étant aromatique ou non,
      - un groupe hétérocyclylalkyle (alkyle de 1 à 4 C), tel que furfurylméthyle ou tétrahydrofurfurylméthyle,
   lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si(R₄R₅) où R₄ et R₅ identiques ou différents représentent un groupe alkyle en C₁ à C₆, ou un groupe phényle.

Des exemples de monomères vinyliques sont le vinylcyclohexane, et le styrène.

Des exemples d'esters de vinyle sont : l'acétate de vinyle le propionate de vinyle, le butyrate de vinyle, l'éthylhexanoate de vinyle, le néononanoate de vinyle, et le néododécanoate de vinyle. Parmi les éthers de vinyle on trouve par exemple le vinyl méthyl ether, le vinyl éthyl éther, et le vinyl isobutyl éther ;
- Les monomères (méth)acryliques ou (méth)acrylamides ou vinyliques à groupe fluoré ou perfluoré, tels que le méthacrylate d'éthyl perfluorooctyle ;
- Les monomères (méth)acryliques ou vinyliques siliconés, tels que le méthacryloxypropyltris(triméthylsiloxy)silane, l'acryloxypropylpolydiméthylsiloxane, ou les (méth)acrylamides siliconés.

Le ou les monomères, à partir duquel ou desquels est susceptible d'être préparée la séquence B, sont, de préférence, choisis parmi les monomères dont la température de transition vitreuse de l'homopolymère correspondant est inférieure ou égale à 110°C, de préférence encore inférieure ou égale à 50°C, encore mieux inférieure ou égale à 20°C.

Les monomères, particulièrement préférés, sont ceux dont la température de transition vitreuse Tg de l'homopolymère correspondant est inférieure ou égale à 50°C, tels que : l'acrylate de méthyle (Tg=10°C), l'acrylate d'éthyle (Tg=-24°C), l'acrylate de n-butyle (Tg=-54°C), l'acrylate de t-butyle (Tg=43°C) l'acrylate d'éthylhexyle (Tg=-50°C), l'acrylate d'isobutyle (-24°C), l'acrylate de méthoxyéthyle (-33°C), le méthacrylate de butyle (Tg=20°C), le méthacrylate d'éthoxyéthyle (Tg=0°C), le méthacrylate de POE (n=8 à 10) (Tg=-55°C), l'acétate de vinyle (Tg=23°C) ; et d'autres monomères, tels que le méthacrylate de méthyle (Tg=105°C), le méthacrylate d'éthyle, le méthacrylate d'isobutyle, l'acrylate de furfuryle, l'acrylate d'isobornyle l'acrylate de tertiobutylcyclohexyle, le styrène, et le vinylcyclohexane.

Les monomères, à partir desquels peut être préparée la séquence B, peuvent être choisis parmi les monomères hydrophiles, incluant les monomères ioniques tels que les monomères cationiques, les monomères anioniques et les bétaïnes ; les monomères non ioniques ; et les monomères pouvant être rendus hydrophiles par suite d'une hydrolyse.

Des exemples de monomères anioniques et cationiques ont déjà été donnés ci-dessus dans le cadre de la description de la séquence A.

Des exemples de monomères ioniques de type bétaïnes sont :
- Les carboxybétaïnes ou sulfobétaïnes éthyléniques obtenues, par exemple par quaternisation de monomères à insaturation éthylénique comportant une fonction amine par des sels de sodium d'acide carboxylique à halogène mobile, par exemple, le chloroacétate de sodium, ou par des sulfones cycliques, par exemple la propanesulfone.

Des exemples de monomères non-ioniques sont :
- les (méth)acrylates d'hydroxyalkyle dont le groupe alkyle a de 2 à 4 atomes de C, en particulier le (méth)acrylate d'hydroxyéthyle ;
- les vinyllactames ;
- les (méth)acrylamides, les (méth)acrylamides de N-alkyle en C₁-C₄, comme l'acrylamide d'isobutyle ;
- et les (méth)acrylates de polysaccharide comme l'acrylate de saccharose.

Les vinyllactames ne peuvent, toutefois, selon l'invention, constituer qu'une proportion minoritaire de la séquence B.

Il est à noter que même si le copolymère comprend une séquence hydrophile, le copolymère global n'est pas forcément hydrophile.

Les copolymères éthyléniques, séquencés, linéaires selon l'invention sont choisis parmi :
- les copolymères biséquencés (AB) ;
- les copolymères triséquencés (ABA, BAB, ABC, ACB), avec C différent de A ou B ;
- les copolymères multiséquencés ayant plus de trois séquences : (AB)ₙ, (ABA)ₙ, (BAB)ₙ, (ABC)ₙ, (ACB)ₙ avec C différent de A ou B ou les copolymères multiséquencés ayant plus de trois séquences différentes, de type ABCD.

Les copolymères selon l'invention peuvent être préparés par polymérisation par voie anionique.

De préférence, toutefois, les copolymères, selon l'invention, sont dans un premier mode obtenus par polymérisation radicalaire contrôlée, et de manière particulièrement préférée, les copolymères selon l'invention peuvent être obtenus par une polymérisation par « ATRP » particulière qui est la technique dite « Reverse ATRP », ou par la technique RAFT, mais les polymères selon l'invention peuvent aussi, selon un second mode, être obtenus par polymérisation radicalaire classique.

### Premier mode

Les copolymères blocs ou séquencés selon l'invention sont de préférence obtenus par polymérisation radicalaire contrôlée, décrite notamment dans "New Method of Polymer Synthesis", Blackie Academic & Professional, Londres, 1995, volume 2, page 1.

La polymérisation radicalaire controlée permet de réduire les réactions de désactivation de l'espèce radicalaire en croissance, en particulier l'étape de terminaison, réactions qui, dans la polymérisation radicalaire classique, interrompent la croissance de la chaîne polymérique de façon irréversible et sans contrôle.

Afin de diminuer la probabilité des réactions de terminaison, il a été proposé de bloquer de façon transitoire et réversible, l'espèce radicalaire en croissance, en formant des espèces actives dites "dormantes" sous forme de liaison de faible énergie de dissociation.

Ainsi, la polymérisation peut être effectuée selon la technique de transfert d'atome (Atom Transfer Radical Polymerization ou « ATRP », en anglais), ou par réaction avec un nitroxyde, ou bien encore selon la technique de "*reversible addition-fragmentation chain* *transfer*" (« RAFT ») ou enfin par la technique de « ATRP » inverse, dite « reverse ATRP ».

La technique de polymérisation radicalaire par transfert d'atomes, aussi connue sous l'abréviation ATRP, consiste à bloquer l'espèce radicalaire en croissance sous forme de liaison de type C-halogénure (en présence de complexe métal/ligand). Ce type de polymérisation se traduit par un contrôle de la masse des polymères formés et par un faible indice de dispersité des masses.

D'une manière générale, la polymérisation radicalaire par transfert d'atomes s'effectue par polymérisation d'un ou de plusieurs monomères polymérisables par voie radicalaire, en présence :
- d'un amorceur ayant au moins un atome d'halogène transférable ;
- d'un composé halogéné comprenant un métal de transition susceptible de participer à une étape de réduction avec l'amorceur et une chaîne polymérique "dormante", celui-ci sera dénommé « agent de transfert de chaîne » ; et
- d'un ligand pouvant être choisi parmi les composés comprenant un atome d'azote (N), d'oxygène (O), de phosphore (P) ou de soufre (S), susceptibles de se coordonner par une liaison σ audit composé comprenant un métal de transition, la formation de liaisons directes entre ledit composé comprenant un métal de transition et le polymère en formation étant évitées.

L'atome d'halogène est de préférence un atome de chlore ou de brome.

Ce procédé est en particulier décrit dans la demande WO 97/18247 et dans l'article de Matyjasezwski et al. publié dans JACS, 117, page 5614 (1995).

La technique de polymérisation radicalaire par réaction avec un nitroxyde consiste à bloquer l'espèce radicalaire en croissance sous forme de liaison de type C-O-NRₐR où R ₐ et R_{b} peuvent être, indépendamment l'un de l'autre, un radical alkyle ayant de 2 à 30 atomes de carbone ou formant l'un et l'autre, avec l'atome d'azote, un cycle ayant de 4 à 20 atomes de carbone, comme par exemple un cycle 2,2,6,6-tétraméthylpipéridinyle. Cette technique de polymérisation est notamment décrite dans les articles « Living free radical polymerization : a unique technique for preparation of controlled macromolecular architectures » CJ Hawker ; Chem. Res. 1997,30,373-82, et "Macromolecular engineering via living free radical polymerizations" publié dans Macromol. Chem. Phys. 1998, vol. 199, pages 923 - 935, ou bien encore dans la demande WO-A-99/03894.

La technique de polymérisation RAFT (reversible addition-fragmentation chain transfer) consiste à bloquer l'espèce radicalaire en croissance sous forme de liaison de type C-S. On utilise pour cela des composés dithio comme les dithioesters (-C(S)S-), tels que les dithiobenzoates, les dithiocarbamates (-NC(S)S-) ou les dithiocarbonates (-OC(S)S-) (xanthates). Ces composés permettent de contrôler la croissance de la chaîne d'une large gamme de monomères. Toutefois, les dithioesters inhibent la polymérisation des esters vinyliques, tandis que les dithiocarbamates sont très faiblement actifs vis-à-vis des méthacrylates, ce qui limite dans une certaine mesure l'application de ces composés. Cette technique est notamment décrite dans la demande WO-A-98/58974 de RHODIA et dans l'article "A more versatile route to block copolymers and other polymers of complexe architecture by living radical polymerization : the RAFT process", publié dans *Macromolecules,* 1999, volume 32, pages 2 071-2 074. La demande WO-A-98/58974 déjà citée et la demande WO-A-99/31144 de CSIRO ont trait à l'utilisation de dithiocarbamates en tant que réactifs « RAFT ». En utilisant ces dithiocarbamates divers monomères sont polymérisés dont l'acétate de vinyle.

Les avantages principaux de la technique « RAFT » sur la technique « ATRP » sont qu'elle ne nécessite pas de catalyseur métallique et également que des amorceurs de radicaux classiques peuvent être utilisés pour amorcer la réaction.

Dans le cas, de la polymérisation radicalaire contrôlée par exemple de la N-vinylpyrrolidone (NVP), des agents de transfert de chaîne préférés utilisés dans le cas de la polymérisation « RAFT » sont les dérivés de diphényldithiocarbamate.

En faisant varier le rapport de la concentration en monomère sur la concentration en agent de transfert de chaîne, la masse moléculaire du polymère peut être modifiée.

La polymérisation se déroule en plusieurs étapes selon le schéma général :
a- Dans une première étape, on effectue la polymérisation du premier monomère ou mélange de monomères pour former un macroamorceur,
b- Les polymères purifiés par précipitation sont séchés sous vide,
c- Ensuite, dans la deuxième étape, on réalise la polymérisation de la seconde séquence constituée par un monomère ou un mélange de monomères, à l'extrémité du macroamorceur (formé à l'étape a).

Les étapes b et c sont répétées autant de fois que nécessaire suivant le nombre de séquences.

Un procédé particulièrement privilégié est le procédé de polymérisation dit « ATRP » inverse (« Reverse ATRP » en anglais) à ne pas confondre avec le procédé de ATRP classique décrit plus haut.

Dans ce procédé de « Reverse ATRP », l'amorçage est réalisé de façon classique par un amorceur capable de donner des radicaux, tels que par exemple l'azobisisobutyronitrile (AIBN) ou un peroxyde, et non plus à l'aide d'un amorceur spécifique.

La présence d'halogénures de métal, tel que CuBr₂ et d'un ligand permet le contrôle de la polymérisation par « capture » réversible des radicaux formés.

Les ligands préférés sont des molécules à base d'amine en particulier la tris(diméthylaminoéthyl)amine (Me₆TREN). Un bon contrôle de la polymérisation est observé en particulier avec le système AIBN/CuBr₂/Me₆TREN.

L'autre procédé privilégié est le procédé de polymérisation, dit « RAFT ».

### Deuxième mode

Les polymères blocs ou séquencés selon l'invention peuvent également être obtenus en utilisant la technique de polymérisation radicalaire classique en effectuant la coulée des monomères de façon séquencée. Dans ce cas, seul le contrôle de la nature des séquences est possible (pas de contrôle des masses).

Il s'agit de polymériser dans un premier temps un monomère M1 dans un réacteur de polymérisation; de suivre, par cinétique, sa consommation dans le temps puis quand M1 est consommé à environ 95% alors d'introduire un nouveau monomère M2 dans le réacteur de polymérisation.

On obtient ainsi un polymère de structure bloc de type M1-M2.

De manière plus précise, un premier procédé de préparation d'un copolymère tel que décrit ci-dessus comprenant au moins une séquence A et au moins une séquence B et éventuellement une ou plusieurs autres séquences différentes des séquences A et B, dans lequel la polymérisation est réalisée par la technique dite de polymérisation radicalaire à transfert d'atomes inverse (« Reverse ATRP ») comprend les étapes successives suivantes :
a) on polymérise le monomère ou les monomères à partir desquels est préparée la séquence A en présence d'un agent de transfert de chaîne, tel qu'un halogénure de métal de transition, d'un amorceur de radicaux, et d'un ligand, et en présence ou non d'un solvant, moyennant quoi, on obtient un macroamorceur ou précurseur fonctionnel capable d'amorcer une polymérisation car comportant à ses extrémités la fonction agent de transfert ;
b) on polymérise le monomère ou les monomères à partir desquels est préparée la séquence B à l'extrémité dudit macroamorceur ou précurseur en présence d'un agent de transfert de chaîne tel qu'un halogénure de métal de transition, d'un amorceur, et d'un ligand, et en présence ou non d'un solvant, moyennant quoi on obtient un copolymère biséquencé de structure A-b-B ;
c) on répète, éventuellement, l'étape b) avec le ou les monomères à partir desquels sont préparées la ou les autres séquences différentes des séquences A et B, moyennant quoi on obtient un copolymère triséquencé ou multiséquencé.

De façon à obtenir des copolymères triséquencés symétriques, il est possible d'utiliser des amorceurs difonctionnels.

Avantageusement, les « agents de transfert de chaîne » sont choisis parmi les halogénures de métaux à l'état d'oxydation le plus élevé, de préférence parmi CuBr₂, CuCl₂, FeCl₂P(phényl)₃, FeCl₃, et RuCl₂P(phényl)₃.

Avantageusement, les amorceurs de polymérisation radicalaire sont choisis parmi :
- les composés azoïques, tels que le 2,2'-azobisisobutyronitrile (AIBN), le 2,2'-azobis(2-butanenitrile), le 4,4'-azobis(4-acide pentanoïque), le 1,1'-azobis(cyclohexanecarbonitrile), le 2-(t-butylazo)-2-cyanopropane, le 2,2'-azobis[2-méthyl-N-(1,1)-bis(hydrox yméthyl)-2-hydroxyéthyl]propionamide, le 2,2'-azobis(2-méthyl-N-hydroxyéthyl) propionamide, le dichlorure de 2,2'-azobis(N,N'-diméthylèneisobutyrami dine), le dichlorure de 2,2'-azobis(2-amidinopropane), le 2,2'-azobis(N,N'-diméthylèneisobutyrami de), le 2,2'-azobis(2-méthyl-N-[1,1-bis(hydroxy méthyl)-2-hydroxyéthyl]propionamide), le 2,2'-azobis(2-méthyl-N-[1,1-bis(hydroxy méthyl)éthyl] propionamide), le 2,2'-azobis(2-méthyl-N-(2-hydroxyéthyl) propionamide), le 2,2'-azobis(isobutyramide)dihydrate ;
- les péroxydes d'hydrogène, tels que l'hydropéroxyde de butyle tertiaire, l'hydropéroxyde de cumène, le t-butyl-peroxyacétate, le t-butylperoxybenzoate, le t-butylperoxyoctoate, le t-butylperoxynéodécanoate, le t-butylpéroxyisobutarate, le peroxyde de lauroyle, le t-amylperoxypivalate, le t-butylperoxypivalate, le peroxyde de dicumyle, le peroxyde de benzoyle ;
- les persulfates alcalins, tels que le persulfate de potassium, le persulfate d'ammonium ;
- les systèmes redox comportant des combinaisons, telles que :
   - les mélanges de peroxyde d'hydrogène, d'alkyle, peresters, percarbonates et similaires et de n'importe lequel des sels de fer, de sels titaneux, formaldéhyde sulfoxylate de zinc ou formaldéhyde sulfoxylate de sodium, et des sucres réducteurs,
   - les persulfates, perborate ou perchlorate de métaux alcalins ou d'ammonium en association avec un bisulfite de métal alcalin, tel que le métabisulfite de sodium, et des sucres réducteurs,
   - les persulfates de métal alcalin en association avec un acide arylphosphinique, tel que l'acide benzène phosphonique et autres similaires, et des sucres réducteurs.

Avantageusement, les ligands sont choisis parmi les ligands tétradentate, tels que le 1,1,4,7,10,10 hémaméthyltriéthylènetétramine (HMTETA), le 1,4,8,11 tétraazacyclotétradécane (cyclame), le 1,4,8,11 tétraméthyl-1,4,8,11-tétraazacyclotétradécane (Me₄ cyclame), et la tris(diméthylaminoéthyl)amine (Me₆TREN) ; les ligands hexadentate, tels que la tétra 2-pyridyl pyrazine (TPPY), la N,N,N',N' tétrabis(2-pyridyl méthyl)éthylène diamine (TPMEDA) ; et la triphénylphosphine (TPP).

Avantageusement, les solvants sont choisis parmi le dioxane, le tétrahydrofuranne (THF), la N-méthylpyrrolidone, l'eau, et leurs mélanges.

Les « agents de transfert de chaîne », amorceurs, ligands, solvants peuvent être identiques ou différents dans l'étape a) et l'étape b), mais de préférence, ils sont identiques. De préférence encore dans la première et la deuxième étapes a) et b), l' « agent de transfert de chaîne » est CuBr₂, l'amorceur est AIBN, le ligand est Me₆TREN, et le solvant est le dioxane.

Ce premier procédé peut permettre de préparer l'un quelconque parmi les copolymères de l'invention, mais il s'applique particulièrement à la préparation d'un copolymère dans lequel la séquence A est un comopolymère issu d'un monomère majoritaire qui est la N-vinylpyrrolidone et d'un monomère minoritaire qui est l'acide acrylique, l'acide méthacrylique, l'acide styrène-sulfonique, l'acide 2-acrylamido 2-méthyl propane sulfonique, ou l'acrylate de tertiobutyle suivi de l'hydrolyse, et la séquence B est un homopolymère issu d'un monomère choisi parmi l'acrylate de méthyle, l'acrylate de t-butyle et le méthacrylate de méthyle.

Un second procédé de préparation d'un copolymère tel que décrit ci-dessus comprenant au moins une séquence A et au moins une séquence B et éventuellement une ou plusieurs autres séquences différentes (C, D) des séquences A et B, dans lequel la polymérisation est réalisée par la technique dite de polymérisation RAFT « Reversible Addition-Fragmentation Chain Transfert » comprend les deux étapes successives suivantes :
a) on polymérise le ou les monomères à partir desquels est préparée la séquence A en présence d'un agent de transfert de chaîne, et d'un amorceur, dans un solvant ou non, moyennant quoi on obtient un macroamorceur ou précurseur comportant à ses extrémités la fonction agent de transfert de chaîne ;
b) on polymérise le ou les monomères à partir desquels est préparée la séquence B à l'extrémité dudit macroamorceur ou précurseur, en présence d'un amorceur, dans un solvant ou non, moyennant quoi on obtient un copolymère A-b-B ;
c) on répète, éventuellement, l'étape b) avec le ou les monomères à partir desquels sont préparées la ou les autres séquences différentes des séquences A et B, moyennant quoi on obtient un copolymère triséquencé ou multiséquencé.

Avantageusement, les agents de transfert de chaîne sont choisis parmi les dithioesters (-C(S)S-), tels que les dithiobenzoates, les dithiocarbamates (-NC(S)S-) et les dithiocarbonates (-OC(S)S-) (xanthates).

De préférence, les agents de transfert de chaîne sont choisis parmi le diphényl dithiocarbamate de malonate de diéthyle (DPCM), le diphényldithiocarbamate d'acétate de fluoroéthyle (DPFEM) et le xanthate de formule C₂H₅OC(S) SCH (CH₃) COOCH₃.

Avantageusement, les amorceurs sont choisis parmi les composés déjà cités ci-dessus pour le premier procédé.

Avantageusement, les solvants sont choisis parmi le dioxane, le tétrahydrofuranne, la N-méthylpyrrolidone, l'eau et leurs mélanges.

Les amorceurs, agents de transfert de chaîne et solvants peuvent être identiques ou différents dans l'étape a) et l'étape b). De préférence, ils sont identiques.

De préférence encore dans la première et la deuxième étape a) et b), l'amorceur est AIBN, l'agent de transfert de chaîne est le diphényl dithiocarbamate de malonate de diéthyle (DPCM), le diphényldithiocarbamate d'acétate de fluoroéthyle (DPFEM) ou le xanthate de formule C₂H₅OC(S)SCH(CH₃)COOCH₃ suivant la nature des monomères à polymériser, et le solvant est le dioxane.

Ce second procédé peut permettre de préparer l'un quelconque parmi les copolymères de l'invention, mais il s'applique particulièrement à la préparation d'un copolymère dans lequel la séquence A est un copolymère issu d'un monomère majoritaire choisi parmi la N-vinylpyrrolidone et/ou la N-vinylcaprolactame et d'un monomère minoritaire qui est l'acide acrylique, l'acide méthacrylique, l'acide styrène sulfonique, l'acide 2-acrylamido-2-méthyl propane sulfonique, ou l'acrylate de tertiobutyle suivi de l'hydrolyse, et la séquence B est un homopolymère issu d'un monomère choisi parmi l'acrylate de méthyle, l'acrylate de t-butyle et le méthacrylate de méthyle.

L'invention concerne également les compositions cosmétiques ou pharmaceutiques comprenant le copolymère de structure spécifique, tel qu'il a été décrit ci-dessus.

Généralement, ces compositions contiennent de 0,1 à 60 % en poids, de préférence de 0,5 à 50 % en poids, et de préférence encore de 1 à 40 % en poids du copolymère selon l'invention.

Ces compositions, par exemple cosmétiques, selon l'invention, comprennent, outre ledit copolymère, un milieu physiologiquement acceptable, c'est-à-dire un milieu compatible avec les matières kératiniques, comme la peau, les cheveux, les cils, les sourcils et les ongles.

De manière générale, il faut considérer que l'ensemble de la composition est physiologiquement acceptable.

Ledit milieu, physiologiquement acceptable, comprend généralement un ou plusieurs solvant(s) approprié(s), physiologiquement acceptable(s), dans lequel le copolymère, selon l'invention, se trouve sous forme dissoute ou dispersée.

La composition, par exemple cosmétique peut ainsi comprendre, en tant que solvant(s) approprié(s) formant une phase hydrophile, un ou plusieurs solvant(s) choisi(s) parmi de l'eau et les mélanges d'eau et de solvant(s) organique(s) hydrophile(s) comme les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols. La phase hydrophile peut, en outre, contenir des éthers en C₂.

L'eau ou le mélange d'eau et de solvant(s) organique(s) hydrophile(s) peut être présent dans la composition selon l'invention en une teneur allant de 0 % à 90 % (notamment 0,1 % à 90 %) en poids, par rapport au poids total de la composition, et de préférence de 0 % à 60 % en poids (notamment 0,1 % à 60 % en poids).

Le milieu physiologiquement acceptable de la composition peut également comprendre, en outre, une phase grasse, notamment constituée de corps gras liquides à température ambiante (25°C en général) et/ou de corps gras solides à température ambiante tels que les cires, les corps gras pâteux, les gommes et leurs mélanges. Ces corps gras peuvent être d'origine animale, végétale, minérale ou synthétique. Cette phase grasse peut, en outre, contenir des solvants organiques lipophiles.

Comme corps gras liquides à température ambiante, appelés souvent huiles, utilisables dans l'invention, on peut citer : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité ; les hydrocarbures linéaires ou ramifiés, d'origine minérale
ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent être présentes en une teneur allant de 0,01 à 90 %, et mieux de 0,1 à 85 % en poids, par rapport au poids total de la composition.

Le milieu physiologiquement acceptable de la composition selon l'invention peut également comprendre un ou plusieurs solvants organiques, cosmétiquement et/ou pharmaceutiquement acceptables (tolérance, toxicologie et toucher acceptables). Ces solvants peuvent être présents généralement en une teneur allant de 0 à 90 %, de préférence de 0,1 à 90 %, de préférence encore de 10 à 90% en poids, par rapport au poids total de la composition, et mieux de 30 à 90 %.

Comme solvants utilisables dans la composition de l'invention, on peut citer les esters de l'acide acétique comme l'acétate de méthyle, d'éthyle, de butyle, d'amyle, de méthoxy-2-éthyle, l'acétate d'isopropyle; les cétones comme la méthyléthylcétone, la méthylisobutylcétone ; les hydrocarbures comme le toluène, le xylène, l'hexane, l'heptane ; les aldéhydes ayant de 5 à 10 atomes de carbone ; les éthers ayant au moins 3 atomes de carbones ; et leurs mélanges.

Les cires peuvent être hydrocarbonées, fluorées et/ou siliconées et être d'origine végétale, minérale, animale et/ou synthétique. En particulier, les cires présentent une température de fusion supérieure à 25 °C et mieux supérieure à 45 °C.

Comme cire utilisable dans la composition de l'invention, on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone.

Les gommes sont généralement des polydiméthylsiloxanes (PDMS) à haut poids moléculaire ou des gommes de cellulose ou des polysaccharides et les corps pâteux sont généralement des composés hydrocarbonés comme les lanolines et leurs dérivés ou encore des PDMS.

La nature et la quantité des corps solides sont fonction des propriétés mécaniques et des textures recherchées. A titre indicatif, la composition peut contenir de 0 à 50 % en poids de cires, par rapport au poids total de la composition et mieux de 1 à 30 % en poids.

Le copolymère peut être associé à un ou des agents auxiliaires de filmification.

Le milieu physiologiquement acceptable pourra donc comprendre, en outre, un ou plusieurs agents auxiliaires de filmification. Un tel agent de filmification peut être choisi parmi tous les composés connus de l'homme du métier comme étant susceptibles de remplir la fonction recherchée, et notamment être choisi parmi les agents plastifiants et les agents de coalescence.

Le milieu physiologiquement acceptable de la composition selon l'invention peut en outre comprendre une ou des matières colorantes choisies parmi les colorants hydrosolubles, et les matières colorantes pulvérulentes comme les pigments, les nacres, et les paillettes bien connues de l'homme du métier. Les matières colorantes peuvent être présentes, dans la composition, en une teneur allant de 0,01 % à 50 % en poids, par rapport au poids de la composition, de préférence de 0,01 % à 30 % en poids.

Par pigments, il faut comprendre des particules de toute forme, blanches ou colorées, minérales ou organiques, insolubles dans le milieu physiologique, destinées à colorer la composition.

Par nacres, il faut comprendre des particules de toute forme irisées, notamment produites par certains mollusques dans leur coquille ou bien synthétisées.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth.

Les colorants hydrosolubles sont par exemple le jus de betterave, le bleu de méthylène.

Le milieu physiologiquement acceptable de la composition selon l'invention peut comprendre en outre en outre une ou plusieurs charges, notamment en une teneur allant de 0,01 % à 50 % en poids, par rapport au poids total de la composition, de préférence allant de 0,01 % à 30 % en poids. Par charges, il faut comprendre des particules de toute forme, incolores ou blanches, minérales ou de synthèse, insolubles dans le milieu de la composition quelle que soit la température à laquelle la composition est fabriquée. Ces charges servent notamment à modifier la rhéologie ou la texture de la composition.

Les charges peuvent être minérales ou organiques de toute forme, plaquettaires, sphériques ou oblongues, quelle que soit la forme cristallographique (par exemple feuillet, cubique, hexagonale, orthorombique, etc). On peut citer le talc, le mica, la silice, le kaolin, les poudres de polyamide (Nylon®) (Orgasol® de chez Atochem), de poly-β-alanine et de polyéthylène, les poudres de polymères de tétrafluoroéthylène (Téflon®), la lauroyl-lysine, l'amidon, le nitrure de bore, les microsphères creuses polymériques telles que celles de chlorure de polyvinylidène/acrylonitrile comme l'Expancel® (Nobel Industrie), de copolymères d'acide acrylique (Polytrap® de la société Dow Corning) et les microbilles de résine de silicone (Tospearls® de Toshiba, par exemple), les particules de polyorganosiloxanes élastomères, le carbonate de calcium précipité, le carbonate et l'hydro-carbonate de magnésium, l'hydroxyapatite, les microsphères de silice creuses (Silica Beads® de Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Le milieu physiologiquement acceptable de la composition selon l'invention peut également contenir des ingrédients couramment utilisés en cosmétique et/ou en pharmacie, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les polymères hydrosolubles, liposolubles ou en dispersion dans l'eau ou une phase grasse, filmogènes ou non, ou leurs mélanges.

Bien entendu, l'homme du métier veillera à choisir ce ou ces éventuels composés complémentaires, et/ou leur quantité, de manière telle que les propriétés avantageuses de la composition correspondante selon l'invention ne soient pas, ou substantiellement pas, altérées par l'adjonction envisagée.

La composition, par exemple cosmétique, selon l'invention peut se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple (notamment de pâte ayant de viscosité dynamique à 25°C de l'ordre de 0,1 à 40 Pa.s sous une vitesse de cisaillement de 200 s⁻¹, après 10 minutes de mesure en géométrie cône/plan). La composition peut être anhydre, par exemple il peut s'agir d'une pâte anhydre.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition selon l'invention peut être une composition de maquillage comme les produits pour le teint (fonds de teint), les fards à joues ou à paupières, les produits pour les lèvres, les produits anti-cernes, les blush, les mascaras, les eye-liners, les produits de maquillage des sourcils, les crayons à lèvres ou à yeux, les produits pour les ongles, tels que les vernis à ongles, les produits de maquillage du corps, les produits de maquillage des cheveux (mascara ou laque pour cheveux).

La composition selon l'invention peut être également un produit capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques ou spray.

Le copolymère selon l'invention lorsqu'il est utilisé dans les produits capillaires tels que les produits pour le maintien de la coiffure ou la mise en forme des cheveux permet d'éviter le poudrage " flaking ".

Les solutions peuvent être conditionnées sous diverses formes : gels, lotions, par exemple, et notamment dans des vaporisateurs, des flacons-pompe ou dans des récipients aérosol afin d'assurer une application de la composition sous forme vaporisée ou sous forme de mousse. De telles formes de conditionnement sont indiquées, par exemple lorsque l'on souhaite obtenir un spray, une mousse pour la fixation ou le traitement des cheveux.

On a vu plus haut que le polymère selon l'invention était particulièrement adapté à une mise en oeuvre dans de telles conditions car il présentait une grande compatibilité avec les gaz propulseurs utilisés notamment dans les récipients aérosols.

Le ou les agents propulseurs peuvent être choisis parmi le diméthyléther, les alcanes en C₃₋₅, tels que le propane, le n-butane et l'isobutane ; le 1,1-difluoroéthane, les mélanges de diméthyléther et d'alcanes en C₃₋₅, et les mélanges de 1,1-difluoroéthane et de diméthyléther et/ou d'alcanes en C₃₋₅.

L'invention va maintenant être décrite, en référence aux exemples suivants, donnés à titre illustratif et non limitatif.

### Exemples

Dans les exemples suivants, on prépare des polymères selon l'invention, par la technique de polymérisation radicalaire par transfert d'atomes inverse, aussi connue sous l'abréviation « Reverse ATRP », ou par la technique de polymérisation « RAFT » (Reverse Addition - Fragmentation Chain Transfer).

De manière générale, la formation de blocs dans le cas du procédé « ATRP » inverse et du procédé « RAFT » se déroule en plusieurs étapes.

Ainsi dans le cas d'un copolymère dibloc, on procèdera par exemple de la manière suivante :
- a/ La première étape consiste en une polymérisation du premier monomère pour former le macroamorceur ou précurseur.
- b/ La seconde étape consiste en la polymérisation du second monomère à l'extrémité du macroamorceur pour former le dibloc.

Entre les deux étapes, une étape de purification peut être nécessaire, en particulier dans le cas de la polymérisation dite « Reverse ATRP ».

On décrira tout d'abord le mode opératoire général suivi dans les exemples pour chacun des procédés : procédé 1 (polymérisation « Reverse ATRP ») et procédé 2 (polymérisation « RAFT »).

### 1. Procédé 1 : « ATRP » inverse

On réalise le procédé de « Reverse ATRP », de préférence avec la tris(diméthylaminoéthyl)amine (TREN Me₆)en tant que ligand, en utilisant CuBr₂ et en mettant en oeuvre AIBN en tant qu'amorceur.

### 1. 1. Matières premières

- Le monomère éthylénique à cycle lactame, tel que la N-vinylpyrrolidone (VP), acquis auprès de la Société ALDRICH®, est distillé sous vide et stocké sous azote à 0°C avant utilisation.
- Les autres monomères, tels que acrylate de tertiobutyle, méthacrylate de méthyle, acrylate de méthyle, acquis auprès de la Société ALDRICH® sont séchés sur hydrure de calcium et distillés sous vide.
- L'amorceur azobisisobutyronitrile (AIBN) est recristallisé dans le méthanol.
- Le CuBr₂ (99,9 %), le CuBr, la poudre de cuivre (99 %) sont acquis auprès de la Société ALDRICH® et utilisés tels qu'ils sont fournis.
- La tris(diméthylaminoéthyl)amine (TREN Me₆) est synthétisée selon les procédures décrites dans la littérature, par exemple dans le document de MATYZASZEWSKI ACS Symp. Ser. 2000 ; 760 ; 207.
- Tous les solvants : THF, dioxane, et DMF sont séchés par distillation sur CaH₂ avant leur utilisation.

### 1. 2. Polymérisation

La procédure générale est la suivante :

### 1. 2. 1. Première étape

### Formation du macroamorceur ou précurseur

CuBr₂ et Me₆ TREN sont mis dans un ballon et on leur additionne du dioxane.

La solution est agitée durant 30 min. à 25°C.

Le ou les monomère(s) (suivant la séquence préparée en premier et formant le macroamorceur) est(sont) additionné(s) puis l'amorceur AIBN.

Le système est soumis à trois cycles de séchage par vide/argon. La solution est ensuite chauffée dans un bain thermostaté.

La viscosité augmente. Après réaction pendant la durée voulue, le système est refroidi à température ambiante.

Le polymère macroamorceur est séparé par précipitation : il est dilué dans le chloroforme puis précipité dans le diéthyléther, cette opération est répétée deux fois. Le complexe de cuivre est séparé du polymère dissous dans le chloroforme par passage sur colonne neutre. La solution de polymère est alors évaporée.

### 1. 2. 2. Deuxième étape

### Polymérisation de la seconde séquence à l'extrémité du macroamorceur

Le macroamorceur obtenu dans la première étape est additionné à une solution de CuBr et de Me₆TREN dans le dioxane sous atmosphère inerte d'azote.

La quantité requise de manomère(s) constituant la seconde séquence est additionnée. La solution est chauffée. Après le temps désiré, le système est refroidi à température ambiante.

Le polymère est séparé par précipitation : il est dilué dans le chloroforme puis précipité dans le diéthyl éther, cette opération est répétée deux fois. Le complexe de cuivre est séparé du polymère dissous dans le chloroforme par passage sur colonne neutre. La solution de polymère est évaporée.

Le polymère est séché.

Le polymère isolé est généralement une poudre blanche.

### 2. Procédé 2 : « RAFT »

On réalise la polymérisation de monomères éthyléniques à cycle lactame, par exemple de vinylactames avec les composés suivants en tant qu'agents de transfert de chaîne :
- diphényldithiocarbamate de diéthylmalonate (DPCM) : 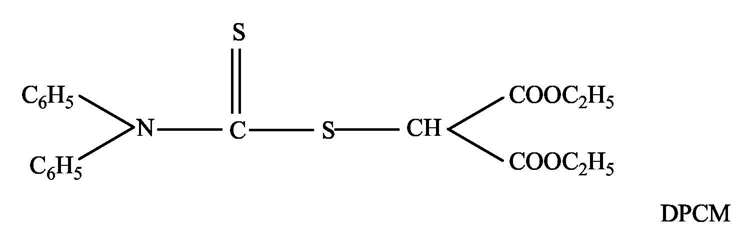
- diphényl dithiocarbamate de fluoroacétate d'éthyle (DPFEM) 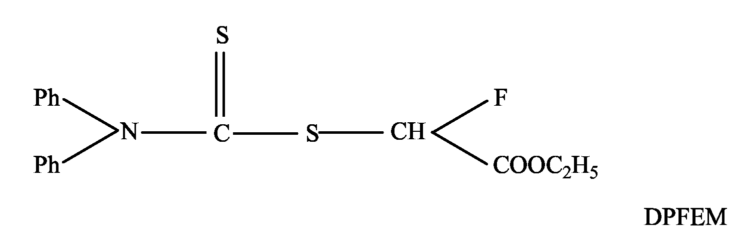
- xanthate : C₂H₅ O C(S)S C H (CH₃) COOCH₃

### Matières premières

- Le monomère éthylénique à cycle lactame tel que la N-vinylpyrrolidone (VP) ou la N-vinylcaprolactame (VCap) est acquis auprès de la Société ALDRICH® et est distillé sous vide avant utilisation.
- Les autres monomères, tel que acrylate de teriobutyle, acrylate de méthyle, méthacrylate de méthyle sont acquis auprès de la Société ALDRICH® et sont séchés sur hydrure de calcium et distillés sous vide.
- L' AIBN est acquis auprès de la Société ALDRICH® et est recristallisé dans le méthanol.
- La diphénylamine, le CS₂, et le bromodiéthylmalonate sont acquis auprès de la Société ALDRICH® et sont utilisés tels qu'ils sont reçus.
- Les solvants tels que le dioxane sont séchés par distillation sur CaH₂ avant utilisation.

### 2. 1. Synthèse de l'agent de transfert de chaîne.

### 2. 1. 1. : Synthèse du diphényldithiocarbamate de diéthyl malonate (DPCM)

Le diphényldithiocarbamate diéthylmalonate (DPCM) est synthétisé à partir de la diphénylamine et du malonate de bromodiéthyle de la manière suivante :

A une solution de 0,625 g de NaH (purifié par lavage avec de l'hexane sec) dans 5 ml de THF (sec), on ajoute à 0°C 1,69 g de diphénylamine (10 mmol) dans 10 ml de DMSO et 5 ml de THF. Le mélange réactionnel est agité pendant 1,5 heures pour donner une solution verte limpide. A cette solution, on ajoute 1,2 éq de CS₂ (1,42 ml, 1,2 mmol), et on agite pendant 30 minutes à 0°C pour obtenir une solution jaune orangée du sel de sodium du diphényldithiocarbamate.

On ajoute du malonate de bromodiéthyle (10 mmol) à la solution ci-dessus à -20°C et on amène lentement la température du mélange réactionnel jusqu'à la température ambiante. Après agitation pendant 2 heures à température ambiante, le mélange réactionnel est traité avec de l'eau et extrait avec de l'éther. La couche éthérée est séchée sur du MgSO₄ et concentrée.

Rendement : 51 %.

La pureté du produit a été vérifiée par RMN.

### 2. 1. 2. Synthèse du diphényl dithiocarbamate d'acétate de fluoroéthyle (DPFEA)

On place de l'hydrure de sodium (7 mmol, 0,168 g, 1,3 eq dans 5 ml THF) dans un flacon séché à la flamme et on l'agite à 0°C. A ce mélange, on ajoute goutte à goutte de la diphénylamine (5,4 mmoles, 1 eq) dans 5 ml de THF, et 10 ml de DMSO et on agite pendant une heure. On ajoute du disulfure de carbone (2,3 eq) à la solution de 0°C et on l'agite pendant encore une demi-heure. On abaisse la température de la solution jusqu'à 18°C et on ajoute l'équivalent d'acétate de fluoroéthyle. Après addition, on élève lentement la température du mélange réactionnel jusqu'à la température ambiante et on l'agite pendant une demi-heure à température ambiante. Le produit obtenu est hydrolysé en ajoutant de l'eau et la couche organique est extraite avec de l'éther. L'extrait éthérée est concentré pour donner des cristaux jaunes de (DPFEA) et la pureté du produit est vérifiée par analyse RMN.

### 2. 2. Polymérisation

2. 2. 1. Le mode opératoire général pour la polymérisation est le suivant que l'agent de transfert de chaîne soit le diphényl dithiocarbamate de diéthylmalonate ou bien le dithiocarbamate d'acétate de fluoréthyle

Dans ce qui suit, la vinylpyrrolidone est mentionnée, mais le procédé de préparation peut être généralisé à tout monomère éthylénique à cycle lactame.

### Première étape

### Préparation du précurseur (ou macroamorceur ): copoly(vinylpyrrolidone / monomère minoritaire)

La polymérisation de la vinylpyrrolidone (VP) et d'un monomère minoritaire (par exemple acrylate de tertiobutyle (séquence A) utilisant le diphényl dithiocarbamate de malonate de diéthyle en tant que réactif RAFT est réalisée en mettant en oeuvre l'AIBN en tant qu'amorceur.

Dans une expérience typique, la vinylprrolidone (VP), le diphényl dithiocarbamate de malonate de diéthyle (rapport agent de transfert de chaîne/monomère = 1/100), l'AIBN (10 % de l'agent de transfert de chaîne) et du dioxane (rapport de 1/1 en volume par rapport au monomère) sont placés dans un tube Schlenk. Le mélange réactionnel est dégazé par trois cycles de congélation-mise sous vide décongélation (« freeze-pump-thaw cycle »), puis fermé hermétiquement sous vide et chauffé dans un bain à température constante à 80°C.

Le macroamorceur « actif » que l'on peut représenter par 'X PVP X avec X= (Phenyl)₂NC(S)S-et X'=-CH(COOC₂H₅)₂ obtenu est purifié par précipitations répétées dans l'éther éthylique et est séché sous vide.

### Deuxième étape

### Polymérisation de la seconde séquence à l'extrémité du macroamorceur poly(vinylpyrrolidone/monomère minoritaire)

La polymérisation du second monomère formant la séquence B (qui est par exemple le méthacrylate de méthyle) a lieu en présence du macroamorceur copolymère poly(vinylpyrrolidone/monomère minoritaire) ci-dessus, dans 1,5 ml de dioxane et en présence de AIBN (0,1 mol % par rapport au total du monomère et du macroamorceur).

Le mélange réactionnel est soumis à trois cycles de congélation - mise sous vide-décongélation et chauffé dans un bain d'huile sous agitation à 80°C pendant 20 heures.

Une fois la réaction terminée, le mélange réactionnel est dissous dans le dichlorométhane (quantité juste nécessaire à la dissolution) et il est précipité dans l'éther.

La solution éthérée trouble est filtrée et concentrée par évaporation instantanée (« flash »)et est ajoutée à du pentane ; le précipité obtenu est séché sous vide à environ 70°C pendant 8 heures.

### 2. 2. 2.

En variante, la polymérisation peut être réalisée de la manière suivante : on effectue d'abord la synthèse d'un macroamorceur poly(méthacrylate de méthyle) (séquence B) par exemple, puis on effectue la polymérisation de la séquence (A) mélange de monomères : vinylpyrrolidone et monomère minoritaire tel que l'acrylate de tertiobutyle à l'extrémité de ce macroamorceur.

On suit le même mode opératoire que dans le paragraphe 2. 2. 1.

### 2. 2. 3. Polymérisation en utilisant un xanthate comme agent de transfert de chaîne : synthèse sans étape de purification intermédiaire (« one pot synthesis » en anglais)

De la N-vinylpyrrolidone (4 mol, 3,74 ; 10⁻² moles) (le mode opératoire peut s'appliquer aussi à un autre monomère éthylénique à cycle lactame), le monomère minoritaire (acrylate de tertiobutyle), l'agent de transfert de chaîne (xanthate ci-dessus, 0,0839 g, rapport agent de transfert de chaîne/monomère=environ 1/100 en moles, et du dioxane (4 ml) sont placés dans un ballon préséché, et de l'AIBN (0,0061 g, 10 % en moles de l'agent de transfert de chaîne) y est ajouté sous azote.

Le mélange réactionnel est soumis à trois cycles de congélation-mise sous vide-décongélation et est chauffé dans un bain d'huile thermostaté à 80°C sous agitation. La réaction est arrêtée après 14 heures par refroidissement dans l'azote liquide.

Le second monomère (formant la séquence B), par exemple l'acrylate de butyle (3 ml), et du dioxane (3 ml) sont ajoutés à ce mélange réactionnel à température ambiante sous azote. Le mélange réactionnel est de nouveau soumis à trois cycles de congélation-mise sous vide-décongélation et est chauffé à 80°C pendant encore 24 heures.

Une fois la réaction terminée, le mélange réactionnel est dissous dans le dichlorométhane (quantité juste nécessaire à la dissolution) et il est précipité dans l'éther. La solution éthérée trouble est filtrée et concentrée par évaporation instantanée (« flash ») et elle est ajoutée à du pentane, le précipité obtenu est séché sous vide à environ 70°C pendant 8 heures.

### Caractérisations

- La conversion est mesurée par pesée du polymère.
- La masse du macroamorceur polyvinyllactame, par exemple copolymère poly(vinylpyrrolidone/monomère minoritaire, est déterminée par chromatographie en phase gazeuse GPC (appareil Varian 9012®) avec des colonnes gels G4000 G3000 G2500 de TSK®, équipées d'un détecteur infrarouge VARIAN® RI 4. L'éluant est un mélange 80/20 eau méthanol contenant 0,1 M de nitrate de sodium. Le débit de l'éluant est de 0,5 ml/min. La calibration est effectuée par des étalons poly(oxyde d'éthylène).
   La mesure de masse n'est donc qu'une mesure comparative et les valeurs ne représentent pas les masses moléculaires réelles. Cependant, les résultats peuvent être utilisés pour noter la tendance dans l'évolution des masses moléculaires et également pour déterminer la dispersité en masse des chaînes.
- La masse du macroamorceur (polyméthacrylate de méthyle, par exemple) est déterminée par GPC (Varian® 9012) avec des colonnes gels G4000 G3000 G2500 de TSK®, équipées d'un détecteur infrarouge Varian® RI 4. L'éluant est le THF. L'étalon est le polystyrène.
- La masse globale (GPC) du copolymère est déterminée par chromatographie GPC en phase solvant (Varian® 9012) avec des colonnes gels G4000 G3000 G2500 de TSK®, équipées d'un détecteur infrarouge Varian® RI 4. L'éluant est le THF. L'étalon est le polystyrène.
- La proportion des différentes séquences est déterminée par RMN¹H (Bruker®, 200 MHz) en faisant le rapport des surfaces des pics correspondants aux monomères des différentes séquences (à savoir par exemple d'une part MMA et d'autre part (VP/comonomère minoritaire). La masse globale (RMN) du copolymère est déduite avec ces valeurs et la masse du macroamorceur déterminée plus haut.

Après avoir décrit ci-dessus le mode opératoire général pour la synthèse des copolymères selon l'invention, on donne ci-après des exemples concernant la préparation d'un copolymère spécifique conforme à l'invention (exemple 1) ainsi qu'un exemple comparatif.

### Exemple comparatif

Dans cet exemple, on prépare un copolymère (non-conforme à l'invention) comprenant une séquence polyvinylpyrrolidone et une séquence poly(méthacrylate de méthyle) : (PVP) 80 %-b-(PMMA) 20 % selon le procédé 1 (« ATRP » inverse) décrit ci-dessus.

### Première étape

### Synthèse du macroamorceur polyvinylpyrrolidone-Br : PVP-Br

Du CuBr₂ et du Me₆TREN sont placés dans un ballon de 50 mL et on ajoute 5 mL de dioxane.

On ajoute ensuite de la vinylpyrrolidone (VP) suivie par de l'AIBN (azobisisobutyronitrile).

Le rapport dioxane/monomère est de 1/1 en volume.

Les proportions AIBN/CuBr₂/CuO/Me₆TREN sont : 1/1,5/0,15/3.

La réaction est réalisée à une température de 80°C pendant 3 heures 45. La conversion atteint 84 %.

### Deuxième étape

### Synthèse du copolymère : Polymérisation du méthacrylate de méthyle (MMA) à l'extrémité du macroamorceur polyvinylpyrrolidone (PVP-Br)

On ajoute le macroamorceur de PVP(PVP-Br) dans du CuBr (rapport 1 :1,5 par rapport au PVP-Br) et du Me₆TREN (1:3 par rapport au CuBr) dans le dioxane sous atmosphère d'azote.

Macroamorceur polyvinylpyrrolidone PVP-Br : 0,026 mM.

Méthacrylate de méthyle (MMA) : 3,87 mM.

La réaction est réalisée à une température de 100°C pendant 3 heures 45.

La conversion est de 60 %.

Les caractéristiques du macroamorceur et du copolymère final sont résumées dans le tableau suivant :

| Copolymère | VP/AIBN | Mn (macroamorceur PVP) (GPC) | % PVP/poids total | % PMMA/poids total | Mn PMMA déduit par RMN |
|---|---|---|---|---|---|
| (PVP) 80 %--b-- (PMMA) 20 % | 1/0,25 | 38 700 | 80,2 % | 19,8 % | 9600 |
| Vinylpyrrolidone = VP | | | | | |
| Azobisisobutyronitrile = AIBN | | | | | |
| Mₙ = masse moléculaire moyenne en nombre | | | | | |

Le polymère obtenu est mis à dissoudre dans de l'eau par ajout d'eau.

Le polymère est insoluble dans l'eau, en effet la solution est laiteuse et instable.

### Exemple 1

Dans cet exemple, on prépare un copolymère (conforme à l'invention) comportant une séquence A (80 % en poids) constituée par un copolymère de 80 % de vinylpyrrolidone (VP) et 20 % d'acide acrylique, et une séquence B (20 % en poids) constituée par du poly(méthacrylate de méthyle). Ce polymère Poly(VP : 80 %/acide acrylique : 20 %) 80 %-b-(PMMA) 20 % résulte de l'hydrolyse de l'acrylate de t-butyle du polymère suivant : poly(VP : 70 %/acrylate de t-butyle : 30 %) 80 %-b-(PMMA) 20 %.

### 1) Synthèse du polymère Poly(VP : 70 %/acrylate de t-butyle : 30 %) 80 %-b-(polyméthacrylate de méthyle) 20 %.

Le polymère recherché est préparé par un procédé analogue à celui décrit dans le paragraphe 2.2.3. ci-dessus. Le mélange de monomères étant adapté pour obtenir le polymère voulu.

On place de la N-vinylpyrrolidone (VP) et de l'acrylate de tertiobutyle (3,74 x 10⁻² moles au total) l'agent de transfert de chaîne (xanthate décrit plus haut, 0,0839 g, rapport agent de transfert de chaîne/monomères : environ 1/100) et du dioxane (4 ml) dans un flacon séché au préalable ; et de l'AIBN (0,0061 g, 10 % en moles par rapport à l'agent de transfert de chaîne) est ajouté dans le flacon sous azote.

Ce mélange réactionnel est soumis à trois cycles congélation-mise sous vide-décongélation et il est chauffé dans un bain d'huile thermostaté à 80°C sous agitation.

La réaction est arrêtée après 14 heures par refroidissement dans l'azote liquide.

Le monomère de la seconde séquence (méthacrylate de méthyle) (3 ml) et du dioxane (3 ml) sont ajoutés à ce mélange réactionnel à température ambiante sous azote. Le mélange réactionnel est de nouveau soumis à trois cycles congélation-mise sous vide-décongélation et est chauffé à 80°C pendant encore 24 heures.

Une fois la réaction terminée, le mélange réactionnel est dissous dans du dichlorométhane (quantité juste nécessaire pour la dissolution) et il est précipité dans l'éther.

La solution éthérée trouble est filtrée et concentrée par évaporation instantanée (« flash ») et elle est ajoutée à du pentane ; le précipité obtenu est séché sous vide à environ 70°C pendant huit heures, et il est caractérisé par GPC et RMN.

Les caractéristiques des réactifs et du procédé sont résumées ci-après.
Agent de transfert de chaîne : xanthate C₂H₅OC(S) SCH (CH₃) COOCH₃.
Rapport molaire : xanthate/monomères : 1/100.
Rapport AIBN/agent de transfert de chaîne xanthate : 10 % en moles.
Rapport monomères/AIBN : 0,1.
Température de la réaction : 80°C.
Solvant : dioxane.
Vinylpyrrolidone (VP) : 0,027 moles, soit 3 g.
Acrylate de tertiobutyle (AtBu) : 0,0098 moles, soit 1,254 g.

Les caractéristiques du copolymère sont résumées dans le tableau suivant :

| Copolymère | VP/AtBu en poids | Mn [poly(VP co AtBu) ] mesurée par GPC | Conver sion du MMA | % PMMA/poids total | Mn PMMA déduit par RMN | % [poly (VP co AtBu)]/poids total |
|---|---|---|---|---|---|---|
| Poly(VP : 70 %/acrylate t-butyle : 30 %) 80 %-b-(polyméthacrylate de méthyle) 20 % | 70/30 | 6 500 | 90 % | 25 | 2 160 | 75 |
| Mn : masse moléculaire moyenne en nombre. | | | | | | |

### 2) hydrolyse du Poly(VP : 70 %/acrylate de t-butyle : 30 %) 80 %-b-(polyméthacrylate de méthyle) 20 %.

Le polymère Poly(VP : 70 %/acrylate de t-butyle : 30 %) 80 %-b-(polyméthacrylate de méthyle) 20 % est dissous dans le dichlorométhane à 10 %. On ajoute à cette solution une solution d'acide trifluoroacétique à raison de 5 équivalents molaires par rapport aux motifs acrylate de t-butyle.

A 10 g de polymère (soit 0,018 mole de AtBu) sont additionnés : 0,09 mole, environ 11 g d'acide trifluoroacétique.

La réaction est effectuée durant 6 h à température ambiante.

Le polymère final selon l'invention Poly(VP : 80 %/acide acrylique : 20 %) 80 %-b-(PMMA) 20 % est alors obtenu.

La solubilité du polymère est appréciée dans le test suivant.

Le polymère préparé ci-dessus est neutralisé par addition d'une solution aqueuse de soude (1M) à raison de 100 % de neutralisation des motifs acide acrylique.

Le polymère selon l'invention se solubilise dans l'eau, au contraire du polymère non-conforme à l'invention de l'exemple comparatif.

Un film est réalisé à partir de la solution.

## Revendications

1. Copolymère éthylénique, séquencé, linéaire comprenant :
- au moins une séquence A susceptible d'être préparée à partir de monomères, constitués de 52 à 99% en poids d'un monomère éthylénique à cycle lactame répondant à la formule (I) suivante : 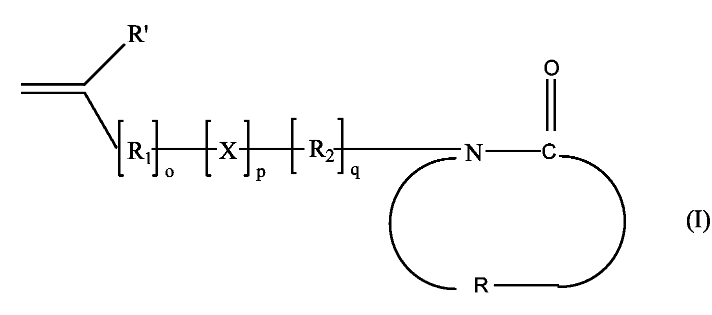 dans laquelle :
• R représente un groupe -(CH₂)ₙ-, où un ou plusieurs des atomes de carbone sont éventuellement remplacés par un atome d'azote ou un atome d'oxygène, où n est un nombre entier de 3 à 12, et où un ou plusieurs des atomes de carbone sont éventuellement substitués par un ou plusieurs groupes alkyle en C₁ à C₆ ;
• R' représente H ou un groupe méthyle ;
• R₁ et R₂, qui peuvent être identiques ou différents représentent un groupe alkylène ou aralkylène, linéaire, ramifié, ou cyclique, de 1 à 22 C, dans lequel un ou plusieurs des atomes de carbone sont éventuellement remplacés par un atome d'oxygène ou d' azote ;
• X est choisi parmi -OCO-, -NHCO-, -COO-, -O- ;
• o, p, q, représentent chacun indépendamment les uns des autres 0 ou 1 ;
- et de 1 à 48% en poids d'au moins un monomère hydrophile ionique ;
- et au moins une séquence B susceptible d'être préparée à partir de monomères ne comportant pas de monomère éthylénique à cycle lactame de formule (I) ou comportant une proportion minoritaire de celui-ci.

2. Copolymère selon la revendication 1, dans lequel dans la formule (I), o est 0, p est 1, q est 1, R₂ représente -CH₂CH₂- ; X représente COO ou CONH, et R est (CH₂)₃ ou (CH₂)₅ ou CH₂CH₂NH.

3. Copolymère selon la revendication 1, dans lequel le monomère éthylénique à cycle lactame est un vinyllactame qui répond à la formule (II) suivante : 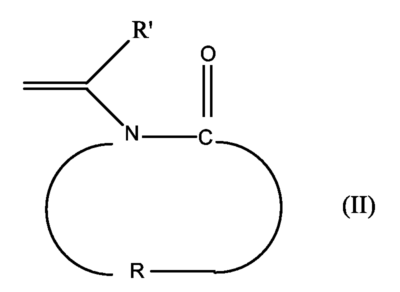 dans laquelle R et R' ont la signification déjà donnée dans la revendication 1.

4. Copolymère selon l'une quelconque des revendications précédentes, dans lequel dans les formules (I) et (II), R représente -(CH₂)ₙ-, où n est un nombre entier de 3 à 5, ou bien R représente -CH₂-CH₂-NH-.

5. Copolymère selon l'une quelconque des revendications précédentes, qui est un copolymère filmogène.

6. Copolymère selon l'une quelconque des revendications précédentes, qui a une masse moléculaire moyenne en nombre du copolymère global de 4 000 à 1 000 000, de préférence de 4 000 à 800 000, de préférence encore de 4 000 à 500 000.

7. Copolymère selon l'une quelconque des revendications précédentes, dans lequel la séquence A, majoritaire en monomère éthylénique à cycle lactame, tel qu'un vinyllactame, représente 50 % en poids ou plus, de préférence de 50 à 99 %, de préférence encore de 55 à 95 %, et mieux de 60 à 90 % en poids du poids total du copolymère.

8. Copolymère selon l'une quelconque des revendications précédentes, dans lequel la proportion de la séquence B est comprise entre 1 et 50 % en poids, de préférence de entre 5 et 45 %, de préférence entre 10 et 40 % en poids du poids total du copolymère.

9. Copolymère selon l'une quelconque des revendications précédentes, dans lequel la masse moléculaire de chaque séquence A ou B est de 2000 à 1 000 000, de préférence encore de 2 000 à 800 000, et mieux de 2 000 à 500 000.

10. Copolymère selon la revendication 1, dans lequel le pourcentage de monomère éthylénique à cycle lactame, tel qu'un vinyllactame, de formule (I) ou (II), dans la séquence A est de 55 à 95 % en poids, de préférence de 60 à 80% en poids, de préférence encore de 65 à 75% en poids, par exemple de 70 % en poids.

11. Copolymère selon l'une quelconque des revendications précédentes, dans lequel ladite séquence A a une température de transition vitreuse globale de la séquence de 0 à 250°C, de préférence de 0 à 220°C, et de préférence encore de 5 à 200°C.

12. Copolymère selon l'une quelconque des revendications précédentes dans lequel ladite séquence A est une séquence hydrophile.

13. Copolymère selon la revendication 1 dans lequel ledit monomère éthylénique à cycle lactame de formule (I) est le pyrrilidinoéthylacrylate, ou le pyrrilidinoéthylméthacrylate.

14. Copolymère selon l'une quelconque des revendications 3 à 12, dans lequel ladite N-vinyllactame de formule (II) est le N-vinylpyrrolidone (n=3), la N-vinylpipéridinone (valérolactame) (n=4), la N-vinylcaprolactame (n=5), la N-vinylimidazolidinone dans laquelle R est le groupe -CH₂-CH₂-NH-, le N-vinyl-5-méthyl-2-pyrrolidone, le N-vinyl-5-éthyl-2-pyrrolidone, le N-vinyl-6-méthyl-2-pipéridone, le N-vinyl-6-éthyl-2-pipéridone, le N-vinyl-7-méthyl-2-caprolactame, ou le N-vinyl-7-éthyl-2-caprolactame.

15. Copolymère selon l'une quelconque des revendications précédentes, dans lequel la séquence A est un copolymère statistique, alterné ou à gradient.

16. Copolymère selon l'une quelconque des revendications précédentes, dans lequel les monomères hydrophiles ioniques sont choisis parmi les monomères cationiques, les monomères anioniques, les bétaïnes, et les monomères pouvant être rendus hydrophiles ioniques par suite d'une hydrolyse.

17. Copolymère selon la revendication 16, dans lequel les monomères hydrophiles ioniques sont choisis parmi les monomères cationiques.

18. Copolymère selon la revendication 16, dans lequel les monomères hydrophiles ioniques sont choisis parmi les monomères anioniques.

19. Copolymère selon la revendication 16, dans lequel les monomères hydrophiles ioniques sont choisis parmi les monomères pouvant être rendus hydrophiles par suite d'une hydrolyse.

20. Copolymère selon la revendication 19, dans lequel les monomères cationiques sont choisis parmi la 2-vinylpyridine ; la 4-vinylpyridine, le (méth)acrylate de diméthylaminoéthyle ; le (méth)acrylate de diéthylaminoéthyle ; le diméthylaminopropyl(méth)acrylamide ; et les formes salifiées ou quaternisées de ceux-ci, qu'il s'agisse de sels d'acides minéraux, tels que l'acide sulfurique ou l'acide chlorhydrique, ou de sels d'acides organiques.

21. Copolymère selon la revendication 18, dans lequel les monomères anioniques sont choisis parmi l'acide acrylique, l'acide méthacrylique, l'acide crotonique, l'anhydride maléique, l'acide itaconique, l'acide fumarique, l'acide maléique, l'acide vinylbenzoïque ; l'acide styrénesulfonique, l'acide 2-acrylamido-2-méthylpropanesulfonique, l'acide vinylphosphonique, le méthacrylate de sulfopropyle, et les sels de ceux-ci.

22. Copolymère selon la revendication 19, dans lequel les monomères pouvant être rendus hydrophiles par suite d'une hydrolyse sont choisis parmi les monomères de type esters (méth)acryliques hydrolysables en acide tels que les (méth)acrylate d'éthyle, de teriobutyle, de benzyle.

23. Copolymère selon l'une quelconque des revendications précédentes, dans lequel la ou les séquences B autres que la séquence A est(sont) susceptible(s) d'être préparée(s) à partir d'un ou de plusieurs monomères éthyléniques choisis parmi : les monomères allyliques, les acrylates, les méthacrylates, les acrylamides, les méthacrylamides, les monomères vinyliques, et leurs mélanges, et éventuellement des monomères éthyléniques à cycle lactame, par exemple des monomères de vinyllactame, de formule (I) ou (II), la proportion en poids desdits monomères (I) ou (II) étant inférieure à 50% en poids, de préférence inférieure ou égale à 45 % en poids, de préférence encore inférieure ou égale à 40 % en poids, mieux inférieure ou égale à 30 % en poids.

24. Copolymère selon l'une quelconque des revendications précédentes, dans lequel le ou lesdits monomères à partir duquel ou desquels la séquence B ou les séquences B est (sont) susceptible(s) d'être préparée(s) est (sont) choisi(s) parmi les monomères suivants :
- Les hydrocarbures éthyléniques de 2 à 10 C, tels que l'éthylène, l'isoprène, ou le butadiène ;
- Les acrylates de formule CH₂ = CHCOOR₃ ;
- Les méthacrylates de formule : 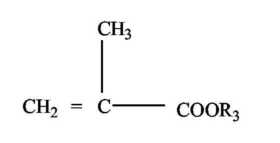 dans lesquelles R₃ représente :
• un groupe alkyle linéaire, ou ramifié, de 1 à 18 atomes de carbone, dans lequel se trouve(nt) éventuellement intercalé(s) un ou plusieurs hétéroatomes choisis parmi 0, N, S et P,
ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆ ou un groupe phényle,
des exemples de ces groupes alkyle sont méthyle, éthyle, propyle, butyle, isobutyle, tertiobutyle, éthylhexyle, octyle, lauryle, et stéaryle,
des exemples de ces groupes dérivés d'alkyle sont les groupes hydroxyalkyle en C₁₋₄ tels que 2-hydroxyéthyle et 2-hydroxypropyle, et les groupes alcoxy (C₁₋₄) alkyle (C₁₋₄) tel que méthoxyéthyle, éthoxyéthyle et méthoxypropyle,
• un groupe cycloalkyle en C₃ à C₁₂, tel que le groupe isobornyle,
• un groupe aryle en C₃ à C₂₀ tel que le groupe phényle,
• un groupe aralkyle en C₄ à C₃₀ (groupe alkyle en C₁ à C₈) tel que 2- phényl-éthyle ou benzyle,
• un groupe hétérocyclique de 4 à 12 chaînons contenant un ou plusieurs hétéroatomes choisis parmi O, N, et S, le cycle étant aromatique ou non,
• un groupe hétérocyclylalkyle (alkyle de 1 à 4 C), tel que furfurylméthyle ou tétrahydrofurfurylméthyle,
lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle, les atomes d'halogène (Cl, Br, I et F), et les groupes Si (R₄R₅), où R₄ et R₅ identiques ou différents représentent un groupe alkyle en C₁ à C₆, ou un groupe phényle,
• des exemples de groupes R₃ sont les groupes méthyle, éthyle, propyle , isobutyle, n-butyle, tertiobutyle isobutyle, héxyle, éthylhéxyle, octyle, lauryle, isooctyle, isodécyle, dodécyle, cyclohexyle, t-butylcyclohexyle, t-butylbenzyle, d'isobornyle, phényle, furfuryléméthyle, tétrahydrofurfurylmethyle, 2-hydroxyéthyle, 2-hydroxypropyle, 2-hydroxybutyle, méthoxyéthyle, éthoxyéthyle, méthoxyéthyle, méthoxypropyle, éthyl-2-perfluorohexyle,
• un autre exemple de groupe R₃ pour les acrylates sont les groupes R₃ = -(OC₂H₄)ₘ-OR'', avec m = 5 à 150 et , R" = H ou alkyle de C₁ à C₃₀, par exemple -POE- méthoxy ;-POE-béhényle ;
- Les (méth)acrylamides de formule : 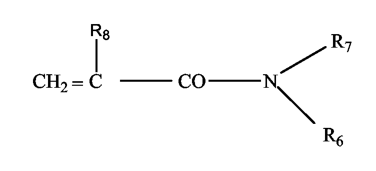 où
R₈ désigne H ou méthyle ;
et R₇ et R₆ identiques ou différents représentent chacun un atome d'hydrogène ou un groupe alkyle de 1 à 18 atomes de carbone linéaire ou ramifié, dans lequel se trouve (nt) , éventuellement intercalé(s) un ou plusieurs hétéroatomes choisis parmi 0, N, S et P, ledit groupe alkyle pouvant, en outre, être éventuellement substitué par un ou plusieurs substituants choisis parmi les groupes hydroxyle, les atomes d'halogène (Cl, Br, I et F), et les groupes Si (R₄R₅), où R₄ et R₅ identiques ou différents, représentent un groupe alkyle en C₁ à C₆, ou un groupe phényle, des exemples de ces groupes sont les groupes méthyle, éthyle, n-butyle, t-butyle, isopropyle, isohexyle, isooctyle, isononyle, et hydroxyalkyle en C₁₋₄ tels que 2-hydroxyéthyle,
• un groupe cycloalkyle en C₃ à C₁₂, tel que le groupe isobornyle,
• un groupe aryle en C₃ à C₂₀ tel que phényle,
• un groupe aralkyle en C₄ à C₃₀ (groupe alkyle en C₁ à C₈) tel que 2- phényl éthyle ou benzyle,
• un groupe hétérocyclique de 4 à 12 chaînons contenant un ou plusieurs hétéroatomes choisis parmi O, N, et S, le cycle étant aromatique ou non,
• un groupe hétérocyclylalkyle (alkyle de 1 à 4 C), tel que furfurylméthyle ou tétrahydrofurfurylméthyle,
lesdits groupes cycloalkyle, aryle, aralkyle, hétérocyclique ou hétérocyclylalkyle pouvant être éventuellement substitué(s) par un ou plusieurs substituants choisi(s) parmi les groupes hydroxyle, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si (R₄R₅), où R₄ et R₅ identiques ou différents représentent un groupe alkyle en C₁ à C₆, ou un groupe phényle,
des exemples de monomères (méth)acrylamide sont le(méth)acrylamide, le N-éthyl(méth)acrylamide, le N-butylacrylamide, le N-t-butylacrylamide, le N-isopropylacrylamide, le N,N-diméthyl(méth)acrylamide, le N,N-dibutylacrylamide, le N-octylacrylamide, le N-dodécylacrylamide, l'undécylacrylamide et le N(2-hydroxypropyl méthacrylamide) ;
- Les composés allyliques de formule :
CH₂ = CH-CH₂-R₉ ou CH₂ = C(CH₃)-CH₂-R₉ ;
- Les composés vinyliques de formule :
CH₂ = CH-R₉,
où R₉ est un groupe
- hydroxyle,
- Cl,
- NH₂,
- OR₁₀, où R₁₀ représente un groupe phényle ou un groupe alkyle en C₁ à C₁₂ (le monomère est un éther de vinyle ou d'allyle),
- Acétamide : NHCOCH₃,
- OCOR₁₁, où R₁₁ représente :
• un groupe alkyle de 2 à 12 C linéaire ou ramifié (le monomère est un ester de vinyle ou d'allyle),
• un groupe cycloalkyle en C₃ à C₁₂ tel que isobornyle, cyclohexyle,
• un groupe aryle en C₃ à C₂₀ tel que phényle,
• un groupe aralkyle en C₄ à C₃₀ (groupe alkyle en C₁ à C₈) tel que 2-phényléthyle ; benzyle,
• un groupe hétérocyclique de 4 à 12 chaînons contenant un ou plusieurs hétéroatomes choisis parmi O, N, et S, le cycle étant aromatique ou non,
• un groupe hétérocyclylalkyle (alkyle de 1 à 4 C), tel que furfurylméthyle ou tétrahydrofurfurylméthyle,
lesdits groupes cycloalkyle, aryle, aralkyle, hétérocyclique ou hétérocyclylalkyle pouvant être éventuellement substitué par un ou plusieurs substituants choisis parmi les groupes hydroxyles, les atomes d'halogène, et les groupes alkyles de 1 à 4 C linéaires ou ramifiés dans lesquels se trouve(nt) éventuellement intercalé(s) un ou plusieurs hétéroatomes choisis parmi 0, N, S et P, lesdits groupes alkyle pouvant, en outre, être éventuellement substitués par un ou plusieurs substituants choisis parmi les groupes hydroxyle les atomes d'halogène (Cl, Br, I et F) et les groupes Si(R₄R₅) où R₄ et R₅ identiques ou différents représentent un groupe alkyle en C₁ à C₆, ou un groupe phényle,
des exemples de monomères vinyliques sont le vinylcyclohexane, et le styrène,
des exemples d'esters de vinyle sont : l'acétate de vinyle le propionate de vinyle, le butyrate de vinyle, l'éthylhexanoate de vinyle, le néononanoate de vinyle ; et le néododécanoate de vinyle,
parmi les éthers de vinyle on trouve par exemple le vinyl méthyl ether, le vinyl éthyl éther, et le vinyl isobutyl éther ;
- Les monomères (méth)acryliques ou (méth)acrylamides ou vinyliques à groupe fluoré ou perfluoré, tels que le méthacrylate d'éthyl perfluorooctyle ;
- Les monomères (méth)acryliques ou vinyliques siliconés, tels que le méthacryloxypropyltris(triméthylsiloxy)silane, l'acryloxypropylpolydiméthylsiloxane, ou les (méth)acrylamides siliconés.

25. Copolymère selon l'une quelconque des revendications précédentes, dans lequel le ou les monomères, à partir duquel ou desquels est susceptible d'être préparée la séquence B, sont choisis parmi les monomères dont la température de transition vitreuse de l'homopolymère correspondant est inférieure ou égale à 110°C, de préférence inférieure ou égale à 50°C, mieux inférieure ou égale à 20°C.

26. Copolymère selon la revendication 24, dans lequel le ou les monomère(s) à partir duquel ou desquels la ou les séquence(s) B est (sont) susceptible(s) d'être séparée(s) est (sont) choisi(s) parmi les monomères suivants dont la température de transition vitreuse Tg de l'homopolymère correspondant est inférieure ou égale à 50°C, tels que : l'acrylate de méthyle (Tg=10°C), l'acrylate d'éthyle (Tg=-24°C), l'acrylate de n-butyle (Tg=-54°C), l'acrylate de t-butyle (Tg=43°C ) l'acrylate d'éthylhexyle (Tg=-50°C), l'acrylate d'isobutyle (-24°C), l'acrylate de méthoxyéthyle (-33°C), le méthacrylate de butyle (Tg=20°C), le méthacrylate d'éthoxyéthyle (Tg=0°C), le méthacrylate de POE (n=8 à 10) (Tg=-55°C), l'acétate de vinyle (Tg=23°C) ; et d'autres monomères, tels que le méthacrylate de méthyle (Tg=105°C), le méthacrylate d'éthyle, le méthacrylate d'isobutyle, l'acrylate de furfuryle, l'acrylate d'isobornyle, l'acrylate de tertiobutylcyclohexyle, le styrène, et le vinylcyclohexane.

27. Copolymère selon la revendication 23, dans lequel le ou lesdits monomères, à partir duquel ou desquels est susceptible d'être préparée la séquence B, est(sont) choisi(s) parmi les monomères hydrophiles.

28. Copolymère selon la revendication 27, dans lequel les monomères hydrophiles sont choisis parmi les monomères ioniques tels que les monomères cationiques, les monomères anioniques et les bétaïnes ; les monomères non ioniques ; et les monomères pouvant être rendus hydrophiles par suite d'une hydrolyse.

29. Copolymère selon la revendication 28, dans lequel les monomères cationiques sont choisis parmi les monomères tels que définis dans la revendication 19.

30. Copolymère selon la revendication 28, dans lequel les monomères anioniques sont choisis parmi les monomères tels que définis dans la revendication 20.

31. Copolymère selon la revendication 28, dans lequel les bétaïnes sont choisis parmi :
- les carboxybétaïnes ou sulfobétaïnes éthyléniques obtenues, par exemple par quaternisation de monomères à insaturation éthylénique comportant une fonction amine par des sels de sodium d'acide carboxylique à halogène mobile, par exemple, le chloroacétate de sodium, ou par des sulfones cycliques, par exemple la propanesulfone.

32. Copolymère selon la revendication 28, dans lequel les monomères non ioniques sont choisis parmi :
- les (méth)acrylates d'hydroxyalkyle dont le groupe alkyle a de 2 à 4 atomes de carbone, en particulier le (méth)acrylate d'hydroxyéthyle ;
- les vinyllactames ;
- les (méth)acrylamides et les N-alkyl (C₁ à C₄) - (méth)acrylamides comme l'isobutylacrylamide ;
- les (méth)acrylates de polysaccharides, comme l'acrylate de saccharose.

33. Copolymère selon la revendication 28, dans lequel les monomères pouvant être rendus hydrophiles par suite d'une hydrolyse sont choisis parmi les monomères tels que définis dans la revendication 22.

34. Copolymère selon l'une quelconque des revendications précédentes, choisi parmi
- les copolymères biséquencés (AB) ;
- les copolymères triséquencés (ABA, BAB, ABC, ACB), avec C différent de A ou B ;
- les copolymères multiséquencés ayant plus de trois séquences : (AB)ₙ, (ABA)ₙ, (BAB)ₙ, (ABC)ₙ, (ACB)ₙ avec C différent de A ou B, ou les copolymères multiséquencés ayant plus de trois séquences différentes, de type ABCD.

35. Composition cosmétique ou pharmaceutique comprenant le copolymère selon l'une quelconque des revendications 1 à 34.

36. Composition selon la revendication 35, contenant de 0,1 à 60 % en poids, de préférence de 0,5 % à 50 % en poids, et de préférence encore de 1 à 40 % en poids du copolymère.

37. Composition selon l'une quelconque des revendications 35 et 36, comprenant outre ledit copolymère un milieu physiologiquement acceptable, dans lequel le copolymère se trouve sous forme dissoute ou dispersée.

38. Composition selon l'une quelconque des revendications 35 à 37, dans laquelle le milieu physiologiquement acceptable comprend un ou plusieurs solvant(s) approprié(s) formant une phase hydrophile choisis parmi l'eau et les mélanges d'eau et de solvant(s) organique(s) hydrophile(s), tels que les alcools et notamment les monoalcools inférieurs linéaires ou ramifiés ayant de 2 à 5 atomes de carbone comme l'éthanol, l'isopropanol ou le n-propanol, et les polyols comme la glycérine, la diglycérine, le propylène glycol, le sorbitol, le penthylène glycol, et les polyéthylène glycols.

39. Composition selon la revendication 38, dans laquelle la phase hydrophile contient, en outre, des éthers en C₂.

40. Composition selon l'une quelconque des revendications 35 à 39, dans laquelle ledit milieu physiologiquement acceptable comprend, en outre, une phase grasse notamment constituée de corps gras liquides à température ambiante et/ou de corps gras solides à température ambiante, d'origine animale, végétale, minérale ou synthétique.

41. Composition selon l'une quelconque des revendications 35 à 40, dans laquelle le milieu physiologiquement acceptable comprend, en outre, un ou plusieurs solvants organiques cosmétiquement et/ou pharmaceutiquement acceptables.

42. Composition selon l'une quelconque des revendications 35 à 41, dans laquelle ledit milieu physiologiquement acceptable comprend, en outre, un ou plusieurs agents auxiliaires de filmification choisis parmi les agents plastifiants et les agents de coalescence.

43. Composition selon l'une quelconque des revendications 35 à 42 dans laquelle le milieu physiologiquement acceptable comprend, en outre, une ou des matières colorantes choisies parmi les colorants hydrosolubles, et les matières colorantes pulvérulentes, comme les pigments, les nacres et les paillettes.

44. Composition selon l'une quelconque des revendications 35 à 43, comprenant, en outre, des charges.

45. Composition selon l'une quelconque des revendications 35 à 44 dans laquelle le milieu physiologiquement acceptable comprend , en outre, un ou plusieurs ingrédient(s) couramment utilisé(s) en cosmétique et/ou en pharmacie, tels que les vitamines, les épaississants, les oligo-éléments, les adoucissants, les séquestrants, les parfums, les agents alcalinisants ou acidifiants, les conservateurs, les filtres solaires, les tensioactifs, les anti-oxydants, les agents anti-chutes des cheveux, les agents antipelliculaires, les agents propulseurs, les polymères hydrosolubles, liposolubles ou en dispersion dans l'eau ou une phase grasse, filmogènes ou non, ou leurs mélanges.

46. Composition selon l'une quelconque des revendications 35 à 45, **caractérisée par le fait qu'**elle se présente sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de pâte, de mousse, de dispersion de vésicules notamment de lipides ioniques ou non, de lotion biphase ou multiphase, de spray, de poudre, de pâte, notamment de pâte souple ou de pâte anhydre.

47. Composition selon l'une quelconque des revendications 35 à 46, **caractérisée par le fait qu'**il s'agit d'un produit capillaire, tel qu'une laque ou un shampooing.

48. Composition selon l'une quelconque des revendications 35 à 46, **caractérisée par le fait qu'**il s'agit d'une composition de maquillage, telle qu'un vernis à ongles.

49. Procédé cosmétique de maquillage ou de soin des matières kératiniques comprenant l'application sur les matières kératiniques d'une composition selon l'une des revendications 35 à 48.

50. Utilisation du copolymère selon l'une quelconque des revendications 1 à 34, pour améliorer la tenue de la coiffure, sans collant notamment au toucher, d'une laque pour cheveux.

51. Utilisation du copolymère selon l'une quelconque des revendications 1 à 34, pour améliorer l'élimination par un shampooing d'une composition capillaire, telle qu'une laque.

52. Utilisation du copolymère selon l'une quelconque des revendications 1 à 34, pour augmenter l'adhérence et la résistance à l'usure, sans collant, notamment au toucher, d'un vernis à ongles.

53. Utilisation du copolymère selon l'une quelconque des revendications 1 à 34, pour optimiser l'adhérence sur la peau, et le confort d'une composition de maquillage.

54. Utilisation du copolymère selon l'une quelconque des revendications 1 à 34 pour diminuer le collant notamment au toucher d'une composition cosmétique.

55. Utilisation du copolymère selon l'une quelconque des revendications 1 à 34 pour diminuer le collant notamment au toucher d'une composition cosmétique dans des conditions d'humidité élevée par exemple de 50 à 100% HR.

56. Utilisation du copolymère selon l'une quelconque des revendications 1 à 34, dans une composition cosmétique telle qu'une composition de maquillage pour masquer les rides qui donne à la peau un aspect lissé, sans tiraillement.
